(19) Europäisches Patentamt
European Patent Office
Office européen des brevets

(11) EP 0 945 144 A2

(12) EUROPÄISCHE PATENTANMELDUNG

(43) Veröffentlichungstag:
**29.09.1999 Patentblatt 1999/39**

(51) Int Cl.$^{6}$: **A61L 15/42**

(21) Anmeldenummer: **99250092.6**

(22) Anmeldetag: **26.03.1999**

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **27.03.1998 DE 19813663**

(71) Anmelder: **Beiersdorf Aktiengesellschaft**
**20245 Hamburg (DE)**

(72) Erfinder:
- **Meyer-Ingold, Wolfgang, Dr.**
  **22457 Hamburg (DE)**
- **Eichner, Wolfram, Dr.**
  **35510 Butzbach (DE)**
- **Ettner, Norbert, Dr.**
  **21629 Neu Wulmstorf (DE)**
- **Schink, Michael, Dr.**
  **22605 Hamburg (DE)**

(74) Vertreter: **UEXKÜLL & STOLBERG**
**Patentanwälte**
**Beselerstrasse 4**
**22607 Hamburg (DE)**

(54) **Wundauflagen zur Entfernung von Störfaktoren aus Wundflüssigkeit**

(57) Die Erfindung betrifft neuartige Wundauflagen, mit denen Störfaktoren des Wundheilungsprozesses gezielt aus der Wundflüssigkeit chronischer Wunden entfernt werden und der normale Heilungsprozeß gefördert wird.

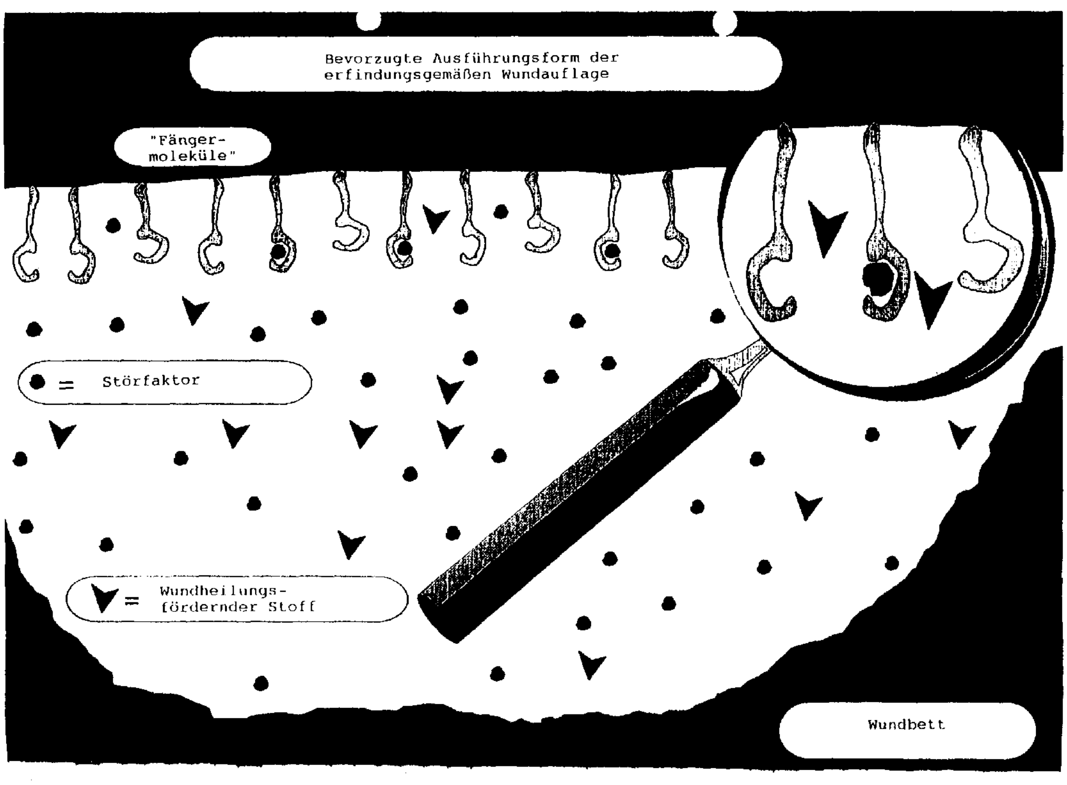


Fig. 1

EP 0 945 144 A2

## Beschreibung

Die Erfindung betrifft neuartige Wundauflagen, mit denen es möglich ist, Störfaktoren des Wundheilungsprozesses gezielt aus der Wundflüssigkeit chronischer Wunden zu entfernen und so den normalen Heilungsprozeß einzuleiten bzw. zu fördern.

Die Behandlung schwer oder nicht heilender Wunden stellt seit jeher ein Problem dar, das bislang nicht befriedigend gelöst werden konnte. In der Vergangenheit wurde durch Aufbringen unterschiedlichster Substanzen und Stoffgemische versucht, die Wundheilung zu fördern. Die Bandbreite topischer Applikationen reichte von Blut, Serum oder Exkrementen über Schwefel, Pech oder Schwermetallsalze bis hin zu Milch, Zucker oder Honig. Heute in der Entwicklung befindliche Wirkstoffe zur topischen Wundbehandlung sind oft gentechnologisch hergestellte körpereigene Substanzen, von denen vermutet wird, daß sie in der chronischen Wunde nicht oder nur in reduzierter Menge vorhanden sind. In einigen Fällen konnte eine im Vergleich zu gesundem Gewebe verringerte Konzentration durchaus nachgewiesen werden.

Ein Beispiel bilden die proliferationsstimulierenden Wachstumsfaktoren (W. Meyer-Ingold, TIBTECH 1993, 11:387). Die Rationale für diese modernen Ansätze ist der Gedanke, daß fehlende oder nicht ausreichende Präsenz solcher Stimuli durch Supplementierung ausgeglichen werden kann und es damit zu einer normalen, ungehindert ablaufenden Wundheilung kommt. Eine Vielzahl von Tierversuchen der letzten Jahre bestätigt diese Annahme. Trotz intensiver Bemühungen ist der klinische Durchbruch - bis auf eine Ausnahme (PDGF) - allerdings noch nicht gelungen. Auch Wirkstoffen wie Peptiden oder Zuckerpolymeren, die sich von den natürlichen Strukturen des Bindegewebes ableiten und die im Tierversuch vielversprechende Resultate zeigten, blieb der klinische Erfolg bislang weitgehend versagt.

Die enttäuschenden Ergebnisse aus der Klinik veranlaßten eine intensive, weltweite Analyse des Targets der topischen Wundtherapeutika, der chronischen Wunde selbst. Das Hauptaugenmerk liegt dabei auf dem als Wundflüssigkeit bezeichneten Exsudat der chronischen Wunde. Obwohl diese Untersuchungen noch am Anfang stehen, zeigen sie doch bereits Unterschiede zwischen chronischer und akuter, d.h. "normaler" Wundflüssigkeit, insbesondere die durch die Bestimmung inflammatorischer Marker (wie z.B. Interleukin-1-alpha und Kollagenase (E.J. Barone et al., Wound Rep. Reg. 1995; 3:374) gestützte Beobachtung, daß der entzündliche Charakter der chronischen Wundflüssigkeit gegenüber der akuten Wundflüssigkeit deutlich erhöht und zudem auch persistierend ist.

Die heutigen Anforderungen an die Funktion von Wundauflagen für chronische Wunden gehen zurück auf G. Winter (Nature 1962; 193:293) und sind jüngst von T. D. Turner neu formuliert (Wound Rep. Reg. 1994; 2:202). Hauptaufgabe ist die Schaffung eines feuchten Wundheilungsmilieus, das im Gegensatz zur traditionellen trockenen Wundbehandlung mit z. B. Mullkompressen den natürlichen Abläufen der Wundheilung physiologische und damit bessere Konditionen bietet. Dabei muß die Wundauflage die Hauptmenge des Exsudates aufnehmen, gleichzeitig aber auf der Wunde selbst einen Flüssigkeitsfilm belassen, in dem die eigentliche feuchte Wundheilung stattfindet. Erreicht werden diese Anforderungen durch gel- oder schwammartige Strukturen und/oder zusätzliche quellfähige oder wasserbindende Substanzen in der Wundauflage. Nach außen sorgt ein atmungsaktiver Film für Sauerstoff- und Wasserdampfdurchlässigkeit und gleichzeitig für eine Barrierefunktion gegenüber möglicherweise aus der Umgebung eindringenden Keimen.

Bemerkenswert ist in diesem Zusammenhang, daß sich bei einer Variation der Eigenschaften der Wundauflagen im Hinblick auf Flüssigkeitsaufnahme oder Wasserdampfdurchlässigkeit z.T. gegenteilige Effekte beobachten lassen, z.B. eine generelle Proteinaufkonzentration (vgl. z.B. V. Achterberg, J. Wound Care, 1996; 5:79). Die selektiven Eigenschaften von Sorbact®, einer durch Kopplung mit Stearinsäure hydrophobisierte Wundauflage aus Cellulose (T. Wadström et al., Acta Pathol. Microbiol. Immunol. Scand. 1985; 93B:359), sind zwar grundsätzlich dazu geeignet, vorzugsweise Bakterien über hydrophobe Wechselwirkungen zu binden wie in EP 0 162 026 und einer klinischen Studie von G. Friman (Current Therapeutic Research 1987; 42:88) beschrieben, eine Differenzierung zwischen unterschiedlichen Bakterienstämmen ist damit aber nicht möglich.

Nachteile der im Stand der Technik bekannten Wundauflagen sind unter anderem die unzureichende heilungsfördernde Wirkung bei chronischen Wunden sowie die o.g. zum Teil gegenteiligen Effekte, die je nach durch unterschiedliche Eigenschaften im Hinblick auf Flüssigkeitsaufnahme oder Wasserdampfdurchlässigkeit der Wundauflagen erzielt werden.

Aufgabe der Erfindung ist es daher, Wundauflagen zur Verfügung zu stellen, die sich in besonderem Maße zur Einleitung bzw. Förderung der Heilung chronischer, d.h. schwer oder nicht heilender Wunden eignen.

Erfindungsgemäß wird die Aufgabe dadurch gelöst, daß man bei der Herstellung der Wundauflagen zusätzliche Substanzen verwendet, die mit in Wundexsudat vorhandenen Störfaktoren, insbesondere endogenen Störfaktoren, wechselwirken, d.h. mit Faktoren, die den Wundheilungsprozeß behindern, wobei man diese zusätzlichen Substanzen kovalent an ein Trägermaterial bindet.

In diesem Zusammenhang werden unter dem Begriff "Störfaktoren" allgemein Stoffe oder Substanzen verstanden, die den Heilungsprozeß von Wunden verhindern oder verlangsamen und damit zur Ausbildung chronischer Wunden führen. Dabei sind im Wundexsudat vorhandene suspendierte Zellen (z.B. entzündliche Zellen wie Leukozyten und Makrophagen oder Bakterien) und Zellfragmente oder gelöste Bestandteile wie Antigene, Radikale (wie z.B. reaktive Sauerstoffspezies, ROS), Ionen (wie z.B. Eisen-Ionen), Proteine, Peptide, Lipide und freie Fettsäuren eingeschlossen.

Unter "endogene Störfaktoren" werden somit in der Wundflüssigkeit vorhandene körpereigene Faktoren verstanden, insbesondere gelöste Bestandteile, die ausschließlich oder zumindest teilweise für eine Heilungsverzögerung verantwortlich sind. Eine bevorzugte Ausführungsform der Erfindung betrifft die Entfernung von sowohl endogenen als auch exogenen (d.h. körperfremden) Störfaktoren.

Bei den Substanzen, die mit diesen Störfaktoren wechselwirken, handelt es sich um Substanzen, die die jeweiligen Störfaktoren selektiv aus dem Wundexsudat entfernen. Im folgenden werden die mit den Störfaktoren wechselwirkenden Substanzen auch als "Fängermoleküle" bezeichnet, wobei diese Bezeichnung nicht als Einschränkung verstanden wird. Je nach Art des Störfaktors kann das Entfernen bzw. Eliminieren auf unterschiedliche Weise erfolgen, d.h auf physikalischem, chemischem oder immunologischem Wege wie zum Beispiel durch Binden, Komplexieren oder Chelatisieren. Im Einzelfall kann der Störfaktor aber auch dadurch beseitigt werden, daß er mit der Substanz (dem "Fängermolekül") chemisch reagiert und in eine Verbindung umgewandelt wird, die die Wundheilung nicht mehr oder nur in vermindertem Umfang behindert. Im Rahmen der vorliegenden Erfindung können die "Fängermoleküle" beispielsweise aus der Gruppe bestehend aus Antikörpern, Chelatoren, Enzyminhibitoren, Enzymen, Enzym-Mimics (vgl. z.B. Dawson et al., Neuroscience Letters 185 (1995) 65-69 - Glutathion-Peroxidase-Mimic; Riley et al., J. Am. Chem. Soc. 116 (1994) 387-388 - Superoxiddismutase-Mimic), Peptiden und anderen Proteinen ausgewählt sein. In jedem Fall ist es von besonderem Vorteil, wenn die an den Träger gebundene Substanz eine hohe Spezifität für den aus der Wundflüssigkeit zu eliminierenden bzw. zu entfernenden Störfaktor aufweist, da auf diese Weise verhindert werden kann, daß für die Wundheilungsprozeß wichtige Substanzen ebenfalls entfernt werden und ein nachteiliger Effekt erzielt wird.

Gegenstand der Erfindung ist demgemäß eine Wundauflage, die dadurch gekennzeichnet ist, daß an ein Trägermaterial Substanzen kovalent gebunden sind, die mit in Wundexsudat vorhandenen Störfaktoren, die den Wundheilungsprozeß behindern, wechselwirken.

Der Vorteil der Erfindung liegt darin, daß durch die kovalent an das Trägermaterial gebundenen Substanzen eine selektive Entfernung der Störfaktoren möglich ist, wobei die "Fängermoleküle" bei Entfernung der Wundauflage ebenfalls entfernt werden ohne daß sie in der Wunde bzw. in der Wundflüssigkeit verbleiben. Auf diese Weise wird zum einen verhindert, daß mit den "Fängermolekülen" weitere Stoffe in die Wunde eingebracht werden, die den Wundheilungsprozeß gegebenenfalls behindern könnten. Zum anderen werden beispielsweise im Falle von Substanzen, die die Störfaktoren, wie z.B. Ionen, Enzyme oder andere Proteine, chelatisieren bzw. in Form eines Komplexes oder auf sonstige Weise binden, die Störfaktoren beim Entfernen der Wundauflage sozusagen aus dem Wundexsudat "herausgezogen". Erfindungsgemäß kann das Wechselwirken auch eine Umwandlung von Störfaktoren in die Wundheilung nicht mehr behindernde Substanzen einschließen.

Die zur Wechselwirkung verwendeten, kovalent an die Wundauflage gebundenen Substanzen werden somit nur vorübergehend in die Wunde eingebracht und werden, nachdem sie ihre bestimmungsgemäße Aufgabe verrichtet haben (d.h. die oben genannte Wechselwirkung eingegangen sind), wieder aus dem Wundbereich entfernt. Gemäß einer bevorzugten Ausführungsform der Erfindung werden dabei gleichzeitig die komplexierten oder an die Fängermoleküle gebundenen Störfaktoren entfernt. Durch diese selektive Entfernung bzw. Eliminierung der Störfaktoren wird der Heilungsprozeß chronischer, d.h. schwer oder nicht heilender Wunden verbessert bzw. eingeleitet.

Im Rahmen der vorliegenden Erfindung ist es somit nicht nur möglich, ein feuchtes Wundmilieu zu generieren oder wachstumsfördernde Substanzen zu applizieren, um den Heilungsprozeß chronischer Wunden zu verbessern, sondern der Heilungsprozeß kann erfindungsgemäß dadurch weiter beschleunigt und gefördert werden, indem in der Wundflüssigkeit vorhandene Störfaktoren spezifisch entfernt bzw. eliminiert werden, z.B. durch selektives Binden an eine Wundauflage oder durch die o.g. Umwandlungsprozesse.

In der entzündlichen Phase der Wundheilung, die der Blutgerinnung und Blutplättchen-Aggregation nach Verletzung und Trauma folgt, wandern bevorzugt Neutrophile und Monozyten in das geschädigte Gewebe ein. Dort beginnen sie mit der Phagozytose von Keimen und dem Abbau von zerstörtem Gewebe und fremden Antigenen. Durch chemische Botenstoffe und Mikroorganismen aktiviert und stimuliert, kommt es zu einer stark erhöhten Produktion von ROS, auch "oxidative burst" genannt. Diese ROS werden in Granula gespeichert und bei weiterer Stimulierung in das extrazelluläre Gewebe in hohen lokalen Konzentrationen zur Bekämpfung von Mikroorganismen freigegeben.

Eine weitere Waffe der Neutrophilen zur Bekämpfung von körperfremden Stoffen sind proteolytische Enzyme wie Elastase, Cathepsin G (J. Travies, American Journal of Med., 1988; 84:37) und Kollagenasen, insbesondere MMP-8 (M. Weckroth et al., J. Invest. Dermatol., 1996; 106:1119). Normalerweise werden sie in zytoplasmatischen Granula gespeichert und gelangen nur kontrolliert in das extrazelluläre Milieu. Durch spontanen Zelltod (Nekrose) der Neutrophilen kommt es zum schnellen Auflösen und Leckschlagen der Zellmembran und somit zum unkontrollierten Ausschütten der proteolytischen Enzyme (C. Haslett und P. Henson, in: The Molecular and Cellular Biology of Wound Repair, 1996; Ed. R. Clark, Plenum Press, New York & London, S. 143). Die Sekretion erfolgt in Form latenter Proenzyme die nach Abspaltung der N-terminalen Propeptide zum Abbau der Bindegewebsproteine Elastin, Kollagen, Proteoglycan (J. Travies, American Journal of Med., 1988; 84:37), Fibronectin (F. Grinell und M. Zhu, J. Invest. Dermatol. 1996; 106:335; S. Herrick et al., Laboratory Investigations 1997; 77:281) und auch Plasmafaktoren wie Fibrin und Antithrombin III (W. Bode et al., EMBO J 1986; 5:2453) führt und somit zur Schädigung des Gewebes und zur Verzögerung der Wundheilung. ROS und Plasmin als Mediatoren inflammatorischer Zellen können die Pro-Matrixmetalloproteinasen (Pro-MMPs) aktivieren (A. Docherty et al. TIBTECH 1992; 10:200) und tragen dabei zu einer weiteren Erhöhung der Proteasekonzentration bei.

In der normal verlaufenden Wundheilung kommt es nach erfolgreicher Beseitigung der auslösenden Reize zur Beendigung der entzündlichen Phase, und der Wiederaufbau des Gewebes kann beginnen. Bleiben jedoch diese Reize bestehen, wandern weitere Leukozyten in das Gewebe nach und werden wiederum aktiviert, was zu einer dauerhaft entzündeten oder chronischen Wunde führt. Die damit einhergende vermehrte Ausschüttung von ROS (O. Senel et al., Annals of Plastic Surgery 1997; 39:516) und proteolytischen Enzymen kann zu einer Freisetzung von Eisen aus geschädigtem Gewebe, den Plasmaproteinen Transferrin und Ferritin (C. Thomas et al., J. Mol. Biol. 1985; 260:3275; P. Biemond et al., Free Radic. Biol. Med. 1988; 4:185), eisenhaltigen Enzymen wie Aconitase (P.R. Gardner et al., J. Mol. Biol. 1995; 270:13399) und anderen Eisen-Schwefel-Proteinen führen (J. Fridovich, Annu. Rev. Biochem. 1995; 64:97). Der Abbau von Ferritin und Transferrin in den aciden, autophagozytischen Vakuolen von Makrophagen durch lysosomale Hydrolasen führt zu einer weiteren Steigerung der Konzentration von ungebundenem Eisen (S. Sakaida et al., Mol. Pharmacol. 1990; 37:435). Dieser transiente, extrazelluläre Pool an freigesetztem Eisen reagiert mit in Wundexsudat gelöstem Sauerstoff zu Eisen-III-Ionen und Superoxid, das rasch in Wasserstoffperoxid dismutiert. Dieses ungeladene Molekül kann im Gegensatz zu Superoxid biologische Membranen durchdringen und im Cytosol an spezifischen Stellen weiterreagieren. Es zerfällt in der als Fenton-Reaktion bezeichneten Umsetzung unter Katalyse durch Eisen-II-Ionen in hochreaktive Hydroxyl-Radikale. In Gegenwart von physiologischen Reduktionsmitteln kommt es zu zyklischen Redoxreaktionen, wobei ROS kontinuierlich erzeugt werden (B. Halliwell und J. Gutteridge, Free Radicals in Biology and Medicine 1989; 2. Auflage, Clarendon Press, Oxford). Unter nicht-pathologischen Bedingungen steht der Eisenmetabolismus unter strikter Kontrolle, und eisenvermittelte Radikalreaktionen der Zelle treten nur limitiert auf.

Die wichtige Rolle von Eisen- und auch Kupfer-Ionen in einer zyklischen Redoxreaktion unter Bildung von ROS (Superoxidanionen, Peroxiddianionen, Singlett-Sauerstoff und Hydroxylradikalen) und damit der oxidativen Schädigung von Biomolekülen und Zellen ist hinreichend dokumentiert. Freies, mobiles Eisen und schwachkomplexiertes Eisen gebunden an Phosporsäureester, organische Säuren und Membranlipide (J. Mutante et al., Agents Actions 1991; 32:167; D.A. Rowley et al., Clin. Sci. 1984; 66:691; B. Halliwell et al., FEBS Lett 1988; 66:691) verursachen z.B. Schädigungen lysosomaler Membranen durch Lipidperoxidation mit einhergehender Ausschüttung von hydrolytischen Enzymen, DNA-und RNA-Modifizierung sowie -Strangbrüche, Degradation von Polysacchariden, Oxidation von Aminosäuren und Spaltung von Enzymen und anderen Proteinen. Daher werden reaktive Sauerstoffspezies außer für die Gewebeschädigungen in schwer heilenden Wunden für viele weitere Prozesse im Körper wie Altern, rheumatoide Arthritis, Arteriosklerose, muskuläre Dystrophie, Parkinsonsche Krankheit oder Autoimmunreaktionen verantwortlich gemacht. Auf den Zusammenhang zwischen Eisen-Ionen, ROS und Gewebeschädigung weisen auch Untersuchungen an Beinulcera hin, bei denen im Gewebe ein erhöhter Eisengehalt und eine reduzierte Menge des endogenen Wachstumsfaktors Transforming Growth Factor $\beta$ (TGF-$\beta$) festgestellt werden konnte. Makrophagen zeigten dabei die größte eisenspezifische Anfärbung, gefolgt von Fibroblasten (Y.J. Francillon et al., Wound Rep. and Regeneration 1996; 4: 240). Durch Phagozytose von seneszenten Erythrozyten und eisenhaltigen Proteinen wird das Eisen in intrazellulären Depots in Form der Eisenspeicherproteine Ferritin und Haemosiderin eingelagert.

Da Wundheilung ein äußerst komplexer biologischer Vorgang ist, innerhalb dessen viele Einzelschritte einer koordinierten Kaskade ablaufen, sind auch die Möglichkeiten einer Störung mannigfach. Zelluläre Störfaktoren wie Bakterien oder ein Übermaß an körpereigenen entzündlichen Zellen wie Leukozyten oder Makrophagen können die Wundheilung ebenso verlangsamen wie aus der Balance geratene regulatorische Proteine oder Peptide vom Typ der Cytokine, wie katabole Enzyme aus den Klassen der Proteasen, Lipasen, Phospholipasen oder Glykosidasen und wie in der Signaltransduktion wirksame Enzyme wie beispielsweise Kinasen. Auch kleine Moleküle oder Ionen sind in ihrer Konzentration in chronischer Wundflüssigkeit verändert, es finden sich beispielsweise reaktive Sauerstoff-Spezies oder Eisen-Ionen erhöht. Erfindungsgemäß hat sich daher gezeigt, daß die Entfernung solcher Störfaktoren dem normalen Wundheilungsprozeß hilft.

Substanzen, die mit Störfaktoren der genannten Art derart in Wechselwirkung treten, z.B. unter Bildung stabiler Komplexe, können entsprechend der Vielfalt der potentiellen Störfaktoren ebenfalls mannigfach sein. Im Rahmen der vorliegenden Erfindung sind die "Fängermoleküle" vorzugsweise Proteine oder organische Moleküle.

Gemäß einer besonderen Ausführungsform der Erfindung kommen spezifische Antikörper gegen ein Cytokin, gegen ein Enzym oder gegen ein regulatorisches Peptid als "Fängermolekül" in Frage. Vorrangig geeignet sind deshalb Antikörper bei der Entfernung der o.g. inflammatorischen Marker, die in der Regel Proteine sind. Für eine möglichst quantitative Entfernung beispielsweise eines definierten Cytokins müssen Antikörper mit hoher Affinität (möglichst größer $10^8$ $mol^{-1}$ ; E. Harlow und D. Lane in: Antibodies - A Laboratory Manual, 1988; S. 514, Cold Spring Harbor Laboratory) gewählt werden, da sonst trotz Einsatzes hoher Antikörper-Konzentrationen keine vollständige Bindung des Störfaktors erfolgen kann.

Weitere, erfindungsgemäß zu verwendende wechselwirkende Substanzen sind Integrine (R. O. Hynes, Cell 1992; 69:11) bzw. allgemein RGD-Peptide (d.h. Peptide, die die Tripeptidsequenz Arginin-Glycin-Aspartat enthalten), die als spezifische Rezeptoren für Zell-Zell- und Zell-Matrix-Interaktionen in immobilisierter Form benutzt werden können, um gezielt eine bestimmte Zellpopulation zu binden.

Analog können Adhäsine zur spezifischen Bindung von bakteriellen Oberflächenstrukturen (C. Heilmann et al., Mol. Microbiology 1996; 20:1083) und damit zur spezifischen Bindung von Bakterien eingesetzt werden (J.M. Higashi et al., J. Biomed. Mater. Res. 1998; 39:341).

Ferner ist der Einsatz von Inhibitoren für Enzyme möglich, wenn die Bildung des entsprechenden Enzym-Inhibitor-Komplexes irreversibel abläuft (sog. "Dead-End-Komplex"). Erfindungsgemäß besonders geeignet sind synthetische niedermolekulare Inhibitoren für proteolytische Enzyme wie z.B. Antipain, Leupeptin, Cystain, Pepstatin, Diisopropylfluorophosphat, 4-(2-Amino-ethyl)-phenylsulfonylfluorid oder Phenylmethansulfonylfluorid. Auch natürliche, proteinogene Protease-Inhibitoren wie z.B. die aus der Klasse der Tissue Inhibitors of Matrixmetalloproteinases oder Aprotinin, Alpha-2-Antiplasmin, Alpha-2-Makroglobulin, Alpha-1-Antichymotrypsin, Sojabohnen Trypsininhibitor und oder Alpha-1-Protease-Inhibitor sind geeignet.

Im Rahmen der vorliegenden Erfindung können als mit den Störfaktoren wechselwirkenden Substanzen auch Enzyme oder Enzym-Mimics als bindendes Prinzip eingesetzt werden, wenn ein korrespondierendes Substrat den Störfaktor darstellt und durch die Reaktion mit dem kovalent an den Träger gebundenen Enzym unschädlich gemacht werden kann. Dieses kann beispielsweise im Fall der ROS erfolgen.

Die Ausnutzung weiterer bekannter biologischer Bindungsphänomene wie beispielsweise die Eigenschaft des Blutproteins Albumin, freie Fettsäuren anzulagern, kann bei Bedarf zu einer Verringerung der Gehalte an freien Fettsäuren in Wundflüssigkeit führen.

Für das selektive Entfernen von störenden Ionen wie z.B. Eisen-Ionen bieten sich spezifische Chelatoren wie z.B. Diethylentriaminpentaessigsäure, N,N'-Bis-(o-hydroxybenzyl)-ethylendiamin-N,N'-diessigsäure, 1,2-Dimethyl-3-hydroxypyrid-4-on, 1,2-Di-methyl-3-hydroxy-3-hydroxypyridin-4-on (B. Porter, Acta Haematol. 1996; 95:13) und Desferrioxamin (Deferoxamin, 30-Amino-3,14,25-trihydroxy-3,9,14,20,25-pentaazatriacontan-2,10,13,21,24-pentaon, DFO) (G.J. Kontoghiorghes, Toxicol. Lett. 1995; 80:1; J.B. Galey et al., Free Radic. Res. 1995; 22:67) an, die Komplexe mit sehr hohen Bindungskonstanten ausbilden. DFO, ein bakterielles Siderophor isoliert aus *Streptomyces pilosus* (H. Bickel et al., Helv. Chim. Acta 1963: 46:1385), und sein Mesilat (Desferal®; DFOM) sind Komplexbildner mit hoher Bindungsaffinität in einem molaren Verhältnis von 1:1 für Aluminium- und Eisen-Ionen. DFO ist aufgebaut aus einem Molekül Essigsäure, zwei Molekülen Bernsteinsäure und drei Molekülen 1-Amino-5-hydroxylaminopentan, die über ihre Hydroxamsäuregruppen mit Eisen-III-Ionen einen stark orangegefärbten Komplex ($K = 10^{-31}$) bilden, der als Ferrioxamin bezeichnet wird (H. Keberle, Ann. N.Y. Acad. Sci., 1964; 119:758). Mit Eisen-II-Ionen kommt es zu einer drastisch verringerten Komplexbildung ($K = 10^{-10}$). Der Schutz, den DFO gegen die Ausbildung von Hydroxylradikalen bildet, liegt im vollständigen Umschließen des gebundenen Eisen-Ions im Gegensatz zu dem Komplexbildner Ethylendiamintetraacetat, der noch die Anlagerung eines leicht austauschbaren Wassermoleküls ermöglicht (E. Graf et al., J. Biol. Chem. 1984; 259:3620). Dadurch wird die Teilnahme des Eisen-Ions in dem als Fenton-Reaktion (Gleichung 1) bezeichneten Reaktionszyklus unter Bildung von reaktiven Hydroxylradikalen unterbunden.

$$Fe^{2+} + H_2O_2 \text{ ----> } Fe^{3+} + OH^- + OH^{\cdot} \qquad (1)$$

Desferrioxamin findet seit über 20 Jahren Verwendung in der Behandlung von schweren Eisenvergiftungen, von Störungen der Eisenspeicherung und von Krankheiten, die zu erhöhten Eisenwerten führen. Beispiele sind idiopathische Hämochromatose, Thalassämie (erblich bedingte Störung der Globinsynthese des Hämoglobins), Sichelzellen-Anämie, gehäufte Bluttransfusionen, übersteigerte Eisenresorption oder durch Medikamente induzierte Hämolysen. Auch in der Wundheilung konnte im Tierexperiment nach systemischer Gabe von DFO eine beschleunigte Wundkontraktion gezeigt werden (H. Cohly et al., Diabetes 1997; 46:295A). Die niedrige Toxizität (J.B. Porter und E.R. Huens, Bailliere's Clin. Haematol. 1989; 2:257) und die geringe Neigung, biologische Membranen zu durchdringen (J.B. Lloyd et al., Biochem. Pharmacol. 1991; 41:1361), machen DFO zu einem idealen Wirkstoff für das Wegfangen überschüssigen Eisens aus extrazellulären Flüssigkeiten. Aus in vitro Experimenten ist bekannt, daß bei Inkubation von gelöstem DFO mit Ferritin und Haemosiderin Eisen bis zur maximalen Sättigung des DFO herausgelöst werden kann. Bei Inkubation mit eisengesättigtem Transferrin können 10 bis 15% Eisen herausgelöst werden, wohingegen das Eisen aus dem Porphyrinring von Haemoglobin und Myoglobin nicht entfernbar ist (H. Keberle, Annu. N.Y. Acad. Sci. 1964; 119:758).

Somit bindet DFO in vivo nur Depoteisen und - zu einem geringen Teil - Transporteisen. Hämoglobin und Myoglobin sowie eisenhaltige Enzyme der Atmungskette werden nicht beeinflußt. Ferner verhindert die hohe Spezifität für Eisen die Eliminierung von anderen für die Wundheilung essentiellen Metall-Ionen wie Magnesium, Calcium und Zink.

Die Kolonisation und Infektion von schwerheilenden Wunden durch Mikroorganismen ist ein großes und oft auftretendes Problem bei der Behandlung von Wunden. Bakterien können eine exzessive Immunantwort auslösen und somit den Verlauf der Wundheilung negativ beeinflußen (M. C. Robson et al., Clinics in Plastic Surgery, 1990, 17:485). Aufgrund von bereits abgestorbenem Gewebe sind chronische Wunden häufiger betroffen als akute (P. Mertz et al., Dermatologic Clinics 1993; 11:739). Zum Beispiel führte eine persistierende Besiedelung eines Beinulcus mit *Staphylococcus aureus* zu einer Vergrößerung des Ulcus unter Ausbildung einer Hämorrhagie und beginnender Nekrose (S. Munk et al., Acta Pathol. Microbiol et Immunol. Scand. 1996;, 104:895; J. Danielsen et al., J. Wound Care, 1997; 6: 308). Die im Serum vorkommenden Eisentransportproteine Transferrin und Lactoferrin - letzteres wird auch von Leukozyten ausgeschieden - weisen durch Eisenentzug der Bakterien eine potente bakteriostatische Wirkung auf und tragen somit zur Bekämpfung von Infektionen bei (R. Critchton, in: Inorganic Biochemistry of Iron Metabolism, 1991; Elis Horwood, New York, 101). Ein an eine Wundauflage immobilisierter, hochaffiner Eisenchelator zeigt eine analoge Wirkung, wobei das Wachstum von Bakterien inhibiert und eine Neukolonisation erschwert werden.

Bezüglich der ROS spielt in aeroben, biologischen Systemen das dimere oder tetramere Enzym Superoxiddismutase (SOD) die Hauptrolle in der zellulären Verteidigung gegen die sauerstoffvermittelte Toxizität von ROS und bei der Regulierung der intrazellulären Sauerstoffkonzentration (I. Fridovich, Annu. Rev. Biochem. 1995; 64:97). Das ubiquitäre Enzym katalysiert die Dismutationsreaktion von Superoxid in das weniger toxische Wasserstoffperoxid und Sauerstoff (Gleichung 2). SOD verhindert ferner die Reaktion von Superoxid mit Stickstoffmonoxid, das für die Regulation des Blutdrucks verantwortlich ist (R.F. Furchgott, Nature 1980; 288: 373). In eukaryontischen Zellen befindet sich die SOD im Zytosol und in den Mitochondrien. Die verschiedenen Enzyme mit Molekulargewichten von 32000 bis 56000 Da haben Metall-Ionen wie Kupfer und Zink (Cu-Zn-SOD), Mangan (Mn-SOD) oder Eisen (Fe-SOD) als Co-Faktoren im katalytischen Zentrum.

$$2\,O_2^{-\cdot} + 2\,H^+ \dashrightarrow H_2O_2 + O_2 \qquad (2)$$

Das durch die Reaktion der SOD gebildete Wasserstoffperoxid wird anschließend durch das in aeroben Organismen ubiquitäre Redoxenzym Katalase in einem mehrstufigen katalytischen Zyklus zu Wasser und Sauerstoff umgewandelt (P. Gouet et al., Nature Structural Biology 1996; 3:951). Außer Katalase sind auch die Enzyme Gluthathion-Peroxidase und Myeloperoxidase befähigt, Wasserstoffperoxid abzubauen. Die weitverbreitetste Form der Katalase ist ein Homotetramer (235000 Da, Rinderleber) mit einer porphyrinischen Gruppe mit einem Eisenatom je Untereinheit.

Erfindungsgemäß werden diese oben genannten Substanzen ("Fängermoleküle") nicht per se appliziert, sondern sie kommen kovalent an eine Wundauflage gebunden zum Einsatz. Durch die Fixierung der "Fängermoleküle" an der Wundauflage ist der Wirkort genau definiert und beschränkt sich auf den Wundbereich bzw. die Wundflüssigkeit. Eine systemische Wirkung, d.h. eine Wechselwirkung mit dem Gesamtorganismus, wird auf diese Weise vermieden. Das Wirkprinzip besteht somit in einer Komplexierung, Chelatisierung oder Bindungsreaktion des Störfaktors oder einer chemischen Reaktion mit dem Störfaktor an der Wundauflage.

Die vorliegende Erfindung betrifft somit neuartige Wundauflagen, die einen zusätzlichen Heileffekt durch die gezielte Entfernung bzw. Eliminierung von Störfaktoren, d.h. von Substanzen, die die Heilung chronischer Wunden behindern, vermitteln.

Die Entfernung bzw. Eliminierung von Störfaktoren kann sowohl auf physikalischem, chemischem oder immunologischem Wege erfolgen. Insbesondere die Bindung bzw. Komplexierung oder Chelatisierung von Störfaktoren stellt eine bevorzugte Ausführungsform der Erfindung dar, da die Störfaktoren mit dem Verbandswechsel endgültig aus dem Wundbereich entfernt werden.

Für die bestimmungsgemäße Funktion eines derartigen Wundverbandes ist die kovalente Bindung der "Fängermoleküle" an die Wundauflage von großer Bedeutung. Deshalb muß das Trägermaterial der erfindungsgemäßen Wundauflage, d.h. das polymere Material (natürlichen oder synthetischen Ursprungs), an das das "Fängermolekül" kovalent gebunden wird, für die Kopplung aktivierbar sein oder, falls das nicht der Fall sein sollte, in einer vorgeschalteten Reaktion aktivierbar gemacht werden. Aktivierbar sind prinzipiell alle Träger aus Materialien, die nach Herstellung der Wundauflage an der Oberfläche noch funktionelle Gruppen wie z.B. -OH, -SH, $-NH_2$, -NHR, -CHO, -COOH oder -COOR tragen. Die dann erforderlichen Kopplungsschritte können chemisch oder enzymatisch erfolgen und sind als allgemeine Immobilisierungstechniken in Standardwerken beschrieben (J.M.S. Cabral und J.F. Kennedey in: Protein Immobilization - Fundamentals and Applications, 1991; S. 73, Ed. R.F. Taylor, Marcel Dekker, New York; Immobilization of Enzymes and Cells, 1997; Ed. G.F. Bickerstaff, Humana Press, Totowa, New Jersey). Es ist jedoch auch möglich, die "Fängermoleküle" bereits an Ausgangsmaterialien zu binden, die für die Herstellung des Trägermaterials bzw. der Wundauflage eingesetzt werden.

Gemäß einer bevorzugten Ausführungsform der Erfindung wird von Polymeren mit aktivierbaren Hydroxylgruppen ausgegangen, die entweder per se als Wundauflage oder als eine von mehreren Komponenten einer Wundauflage verwendet werden.

Erfindungsgemäß kommen beispielsweise folgende Trägermaterialien in Frage: natürliche sowie modifizierte und andere Formen von Cellulose [wie z.B. Carboxymethylcellulose, Celluloseacetat und Viskose-Fasern, bakterielle Cellulose (U. Geyer et al., Int. J. Biol. Macromol. 1994; 16:343)], Alginate, Hyaluronsäure, Chitin oder Chitosane sowie andere Polysaccharide wie z.B. Sephadex®, Sephacryl®, Sepharose®, Superdex®, synthetische Polymere wie z.B. Polyamide, Polyester, Polyolefine, Polyacrylate, Polyvinylalkohole, Polyurethane und Silicone einschließlich deren Mischungen und Copolymere, in die durch Coronabehandlung, Plasmabehandlung oder Polymergrafting (chemical grafting) verschiedene funktionelle Gruppen wie z.B. Hydroxyl, Carbonyl, Carboxyl, Amino oder Peroxyl oberflächig eingeführt werden können (D.M. Coates und S.L. Kaplan, MRS Bulletin 1996; 8:43). Die o.g. Trägermaterialien können als Filme, Gele, Schäume, Laminate und Komposite (Mischungen) eingesetzt werden.

Die Aktivierung der funktionellen Gruppe zur kovalenten Kopplung von Proteinen, Antikörpern, Enzymen oder niedermolekularen organischen Substanzen über Aminogruppen ist in Standardwerken wie z.B. W.H. Scouten, Immobilized Enzymes and Cells, in: Methods Enzymol., Ed. K. Mosbach, 1987; 135:30 und in: Immobilization of Enzymes and Cells, 1997; Ed. G.F. Bickerstaff, Humana Press, Totowa, New Jersey, ausführlich beschrieben. Als funktionelle Aminogruppen bei Proteinen, Antikörpern und Enzymen können die Aminogruppe des Aminoterminus und/oder die ε-Aminogruppen der oberflächenexponierten Aminosäureseitenketten von Lysin und Arginin verwendet werden. Zur Aktivierung von Hydroxylgruppen die sich an einem löslichen Polymer befinden, können Reagenzien wie beispielsweise 1,1'-Carbonylditriazol, 1,1'-Carbonyldiimidazol (CDI), Cyanurchlorid, Chlorameisensäureester, Succinanhydrid, Dicyclohexylcarbodiimid, p-Nitrophenylchloroformiat, 2,4,5-Trichlorophenylchloroformiat, p-Toluylchlorid und Tresylchlorid eingesetzt werden (vergleiche C. Delgado et al. Critical Reviews in Therapeutic Drug Carrier Systems, 1992; 9:249).

Entsprechende Kopplungsverfahren sind im Stand der Technik bekannt und werden z.B. bei der Herstellung von Stoffen für präparative und analytische Anwendungen eingesetzt. So ist beispielsweise in der EP 0 087 786 ein Verfahren zur Immobilisierung des Eisen-Chelators DFO über Bindung der primären Amingruppe beschrieben, wobei DFO an Agarose-Polyaldehyd-Gelperlen gebunden wird. Die Aktivierung der Agarose war zuvor durch Periodat-Oxidation erfolgt. Die kovalente Bindung von DFO an gelöste Bio-polymere wie Dextran, Hyaluronsäure, Hydroxyethyl-Stärke, Methylcellulose, Inulin und humanes Serumalbumin ist ferner in EP 0 304 183 beschrieben. Dabei wird die terminale Aminfunktion des DFO mit einer Aldehydgruppe des Polysaccarids zu einer Schiffschen Base gekoppelt und anschließend mit Natriumborhydrid reduziert wie von S. Margel und E. Wiesel (J. Pol. Chem. Sci., 1984; 22:145) und in EP 0 087 786 beschrieben. Auch andere Arzneistoffe oder Proteine lassen sich an Agarose-Polyaldehyd-Gelperlen kovalent über die Reaktion primärer Amin- oder Thiolgruppen mit den Aldehydgruppen koppeln. WO 96/39125 beschreibt eine Matrix bestehend aus einer hochviskosen kubischen Phase aus z.B. amphiphilischem Glycerylmonooleat, in der Rettichperoxidase oder Katalase eingebettet ist. Diese Matrix stellt eine Barriere zwischen dem auf diese Weise eingebundenen Enzym und externen proteolytischen Enzymen oder zellulären Verteidigungssystemen des Patienten dar und verhindert so eine Freisetzung des Enzyms. Die katalytische Aktivität des Enzyms bleibt für das jeweilige Substrat durch Poren und Kanäle in der Matrix zugänglich. M.M. Hossain und D.D. Do (Biotechnology & Bioengineering, 1988; 31:730) beschreiben die Immobilisierung des Enzyms Katalase an Glaspartikel mit kontrollierter Porengröße und anschließende kinetische Messungen. Die kovalente Modifizierung von SOD (P. Pyatak et al., Res. Com. Chem. Path. and Pharmacol., 1980; 29:115), und KAT (A. Abukovski et al., J. Biol. Chem., 1976; 252:3582) und anderen Enzymen (M. L. Nucci et al., Advanced Drug Delivery Reviews, 1991; 6:133) mit Polyethylenglykol durch Kopplung von aktiviertem Polyethylenglykol an Aminogruppen der Enzyme ist beschrieben. H. Hirano et al. (J. Controlled Release, 1994; 28:203) beschreiben die Herstellung von SOD-Polymerkonjugaten mit aktiviertem Divinylether und Maleinsäureanhydrid. WO 95/15352 beschreibt die kovalente Einbindung von Peroxidase und Katalase über Aminogruppen in ein Polymergel bestehend aus dem Protein BSA und voraktiviertem Polyethylenglykol. G. Maneke und D. Polakowski (J. Chrom., 1981; 215:13) beschreiben die Immobilisierung von α-Chymotrypsin an eine Polymermatrix aus Polyvinylalkohol und Terephthalaldehyd.

Im Stand der Technik ist bislang nicht beschrieben, Substanzen, die mit in Wundexsudat vorhandenen Störfaktoren, die den Wundheilungsprozeß behindern, wechselwirken, zur Herstellung von Wundauflagen zu verwenden. Durch analoge Anwendung der oben genannten Kopplungsverfahren werden erfindungsgemäß die mit dem Störfaktor wechselwirkenden Substanzen durch chemische Reaktion kovalent an für Wundauflagen geeignete Trägermaterialien gekoppelt und gezielt neuartige Wundauflagen zur selektiven Entfernung von Störfaktoren aus Wundflüssigkeit schwer oder nicht heilender Wunden hergestellt. Durch die vorliegende Erfindung werden erstmals neuartige Wundauflagen zur Verbesserung des Heilungsverlaufes dieser Wunden zur Verfügung gestellt.

Als Wundauflagen kommen beispielsweise Wundauflagen aus der Gruppe bestehend aus Verbänden, Verbandmull, Binden, Kompressen, Watten, Pflastern, Folien, Filmen, Hydrokolloidverbänden, Gelen und dergleichen in Frage.

Im Rahmen der vorliegenden Erfindung können somit die im Stand der Technik bekannten Wundauflagen verwendet werden und erfindungsgemäß durch kovalentes Binden von "Fängermolekülen" modifiziert werden. So können insbesondere Wundauflagen verwendet werden, die Feuchtigkeit aufnehmen. Ferner ist es selbstverständlich möglich, mit den erfindungsgemäßen Wundauflagen gleichzeitig wundheilungsfördernde Stoffe, wie z.B. Wachstumsfaktoren (z.B. PDGF) regulatorische Cytokine, Peptide und Hormone, in die Wunde zu applizieren, die geeignet sind, die Wundheilung zu fördern bzw. den Heilungsvorgang zu beschleunigen.
Die vorliegende Erfindung wird nachfolgend anhand von Beispielen und Figuren erläutert:

<u>Beschreibung der Figuren:</u>

Figur 1 ist eine schematische Darstellung einer besonderen Ausführungsform der Erfindung, in der das selektive Binden der Störfaktoren an die Fängermoleküle gezeigt wird.

Figur 2 ist eine graphische Darstellung des kinetischen Verlaufs der Komplexierung von gelöstem Eisen durch an medizinische Verbandsbaumwolle immobilisiertes DFO. Innerhalb der ersten 2 h wird der Großteil des angebotenen Eisens durch die DFO-Wundauflage gebunden. Nach 2 h Inkubation werden 75 %, nach 24 h Inkubation werden 92 % des angebotenen Eisens aus der Lösung komplexiert. Die Eisenbindungskapazität der eingesetzten DFO-Cellulose-Muster beträgt nach Übernachtinkubation maximal 38 µmol Eisen/g Muster. Die nicht aktivierten bzw. völlig unbehandelten Cellulose-Kontrollen und die reine Eisenlösung zeigen keine signifikante Eisenabnahme im Überstand.

Figur 3 ist eine graphische Darstellung des zeitabhängigen Verlaufs der Komplexierung von gelöstem Eisen durch an Polyurethan immobilisiertes DFO. Nach Übernachtinkubation wird eine Eisenbindungskapazität von 17 µmol Eisen/g Polyurethan erreicht. Die mitgeführten Kontrollen (unbehandeltes und nicht-aktiviertes Polyurethan) zeigen kein signifikantes Eisenbindungsverhalten.

Figur 4 zeigt die selektive Bindung von PDGF-AB mit einer Konzentration von 50 ng/ml durch an Cellulose immobilisierte anti-PDGF-Antikörper.

**<u>Beispiele:</u>**

<u>Beispiel 1 - Immobilisierung von DFO an Cellulose</u>

Die Experimente zur Immobilisierung von Desferrioxamin (DFO) an Cellulose wurden wie folgt durchgeführt: 5 g einer medizinischen Verbandsbaumwolle (Gazomull®, Beiersdorf AG) wurden 30 min in 100 mM Bicarbonatpuffer gekocht, anschließend mit 2 l Wasser gespült, an der Luft getrocknet und schließlich durch Zugabe von trockenem Aceton entwässert und in einem Vakuumkonzentrator (Bachhofer, Reutlingen) 60 min auf Uhrgläsern bei Raumtemperatur getrocknet. Anschließend wurde die Verbandsbaumwolle mit 5 g 1,1'-Carbonyldiimidazol (CDI; Fluka, Buchs) (in 500 ml trockenem Aceton frisch angesetzt) für 60 min unter Rückfluß bei 60°C aktiviert (1 %ige CDI-Lösung). Überschüßiges CDI wurde durch Waschen mit Aceton entfernt. In nachfolgenden Transferbädern mit Aceton-Wasser-Gemischen von 3:1, 1:1 und 1:3 und Trocknen an der Luft wurde das Aceton aus der Verbandsbaumwolle entfernt. Die Kopplungsreaktion von DFO an die aktivierte Verbandsbaumwolle wurde in einer Glassäule (Pharmacia, Uppsala) mit den Dimensionen 2.6 cm x 40 cm durchgeführt. Dazu wurden 3.28 g Desferrioxaminmesilat (DFOM) (Sigma, München) in 500 ml Bicarbonatpuffer (100 mM, pH 8.5) gelöst (was eine Konzentration von 10 mM ergab) und mit einer peristaltischen Schlauchpumpe (Ismatec, München) im Gegenstromprinzip von unten nach oben bei einer Flußrate von 10 ml/min im Kreislauf durch die Säule geleitet. Gekoppelt wurde für 18 h bei Raumtemperatur unter Alufolienummantelung der Glassäule. Durch mehrmaliges Waschen mit Bicarbonatpuffer (25 mM, pH 8.5) wurde nichtgebundenes DFO aus der Verbandsbaumwolle entfernt. Abschließend wurde das Cellulose-DFO-Stück luftgetrocknet.
Die Cellulose-DFO-Addukte wurden wie unten beschrieben hinsichtlich der Menge an immobilisiertem DFO untersucht und charakterisiert.
Die Quantifizierung der an Verbandsbaumwolle immobilisierten DFO-Menge mit HPLC und Spektroskopie ergab Werte von 30 bis 50 µmol/g. Immobilisiertes DFO wurde mit Eisen gesättigt (als DFO-Fe bezeichnet), und die Freisetzung von Eisen aus DFO-Fe wurde mit Gallium-III unter reduktiven Bedingungen zu 5 bis 10 µmol/g bestimmt. Die Eisenbindungsaktivität wurde durch Inkubation für 2 Stunden eines 50 mg Musters in 30 ml einer Lösung bestehend aus 0.2 mM $FeSO_4$ (120 µmol/g), 1 mM $MgCl_2$, 1 mM $CaCl_2$ und 154 mM NaCl mit einem pH-Wert von 5.6 durchgeführt. Es zeigte sich eine Eisenaufnahme der angebotenen Eisenmenge von 38 µmol/g (32 %) im Vergleich zu 4 µmol/g (3 %) von unbehandelter Cellulose und nicht-aktivierter Cellulose/DFO als Kontrolle.

Beispiel 2 - Immobilisierung von DFO an einen Celluloseschwamm

500 mg eines gepreßten Stücks aus regenerierter Cellulose (Cellspon®, Cellomeda Oy, Turku) mit einer Dicke von 2 mm wurden 30 min in Bicarbonatpuffer (120 mM, pH 8.5) gekocht, mit Wasser gespült, an der Luft getrocknet, anschließend mit absolutem Aceton entwässert und im Vakuum getrocknet. Die Aktivierung mit 500 mg (3.08 mmol) CDI in 50 ml absolutem Aceton wurde in einem 100 ml Glaskolben bei 60°C für 60 min unter Rückfluß durchgeführt. Überschüßiges CDI wurde durch Waschen mit Aceton entfernt und dieses wiederum mit Aceton-Wasser-Transferbädern (s. o.) und durch Trocknen an der Luft. Die Kopplung mit 250 mg (0.38 mmol) DFOM erfolgte in 40 ml Bicarbonatpuffer (100 mM, pH 7.7) in 50-ml-Schraubgefäßen (Greiner, Nürtingen) unter intensiver Durchmischung. Nach 18 h wurde die Kopplungsreaktion gestoppt und mehrmals mit 100 mM, 500 mM Bicarbonatpuffer und Wasser gewaschen bis kein DFO aus dem Cellspon®-Stück nachweisbar war. Zuletzt wurde das mit DFO gekoppelte Cellspon®-Stück an der Luft getrocknet und in Schraubgefäßen bei Raumtemperatur aufbewahrt.

Die Messung der an Cellspon® immobilisierten DFO-Menge mit HPLC und Spektroskopie (s.u.) ergab Werte von 35 bis 70 µmol/g. Die Freisetzung von Eisen aus DFO-Fe zeigte Werte von 15 bis 40 µmol/g. Die Eisenbindungsaktivität wurde wie in Beispiel 1 beschrieben durchgeführt. Es zeigte sich eine mit Verbandsbaumwolle vergleichbare Eisenaufnahme.

Beispiel 3 - Immobilisierung von DFO an Levagel

6.4 g (1 mmol) Levagel (Bayer, Leverkusen), ein Copolymer aus Propylenoxid und Ethylenoxid mit einem Molekulargewicht von 6400 g/mol, wurden in 50 ml absolutem Aceton unter Rühren in einem Rundkolben unter Stickstoff-Schutzgas gelöst. Dazu wurden 160 mg (1 mmol) CDI ebenfalls gelöst in 50 ml absolutem Aceton in einem Tropftrichter über eine Zeit von 20 min zugegeben. Nach 60 min wurde das Lösungsmittel in einem Rotationsverdampfer (Heidolph, Kehlheim) abdestilliert und das CDI-aktivierte Levagel sofort weiter umgesetzt. Für die Kopplungreaktion wurden 250 mg (40 µmol) CDI-aktiviertes Levagel in Bicarbonatpuffer (250 mM, pH 8,5) mit 30% Ethanol gelöst, da Levagel in wässrigen Systemen eine Emulsion bildet. 87.5 mg (135 µmol) DFOM wurden in 13.5 ml Bicarbonatpuffer gelöst und 12 h bei Raumtemperatur gerührt. Das Reaktionsgemisch wurde anschließend in einen Dialyseschlauch (Spektrapor, Serva) mit einem Ausschlußvolumen von 3500 Da eingefüllt und zweimal für 8 h gegen je einen Liter Phosphatpuffer/Ethanol dialysiert. Aufgrund der Molekülgröße können nur niedermolekulare Substanzen wie Imidazol, Salze und nichtreagiertes DFO die Poren der Dialysemembran passieren, wogegen Levagel und Levagel-DFO in der Membran bleiben.

Beispiel 4 - Immobilisierung von DFO an eine Polyurethangel

Die Polyurethangele (PU-Gel) bestehen aus einer Levagelkomponente und einer Diisocyanatkomponente wie beispielsweise Hexamethylendiisocyanat, Hexahydro-1,3- und/oder 1,4-Phenylendiisocyanat, Perhydro-2,4'- und/oder -4,4'-toluyldiisocyanat mit einem NCO/OH-Verhältnis von 0.3 bis 0.7. Da nicht alle OH-Funktionen während des Vernetzungsprozesses abreagieren, können auf der Oberfläche der Polyurethangel die gleichen Aktivierungs- und Kopplungs-Reaktionen ablaufen wie an freiem Levagel.

Unter Ausschluß von Feuchtigkeit wurde ein PU-Stück von 14 g (5 x 5 cm, 0,5 cm Dicke), was in etwa 2.2 mmol an Levagel entspricht, in 75 ml absolutem Aceton in einen Glaskolben eingelegt und mit 355 mg (2.2 mmol) CDI versetzt. Das PU-Stück wurde unter Rückfluß bei 50°C unter Rühren und Luftabschluß für 40 min aktiviert. Dabei quoll das PU-Stück um ca. 50% durch Eindringen von Aceton und darin gelöstem CDI auf. Nach der Aktivierungsreaktion wurde das PU-Stück mehrmals kurz mit Aceton gewaschen, um überschüßiges CDI und entstandenes Imidazol zu entfernen. Danach wurde das PU-Stück für 1 h in einem Vacuumkonzentrator gelegt und unter Schrumpfen das Lösungsmittel Aceton entzogen. Das CDI-aktivierte PU-Gel wurde sofort mit 1.4 g DFOM (2.2 mmol) in 75 ml Bicarbonatpuffer (100 mM, pH 8.5) versetzt und 18 h bei Raumtemperatur gerührt. Anschließend wurde das gebildete PU-DFO-Gel 3 mal mit Bicarbonatpuffer (25 mM, pH 8.5) gewaschen, an der Luft getrocknet und in einer mit Parafilm (American Can Company, Greenwich) verschlossenen Glasschale bei 4°C aufbewahrt.

Die Quantifizierung der an ein PU-Gel immobilisierten DFO-Menge mit HPLC und Spektroskopie ergab Werte von 45 µmol/g. Die Freisetzung von Eisen aus PU-DFO-Fe wie unten beschrieben wiesen Kopplungsausbeuten von 8 µmol DFO/g auf, im Gegensatz zu nichtaktiviertem PU-Gel, dessen freigesetzte Eisenmenge bei 0.2 µmol DFO/g lag. Die Eisenbindungskapazität wurde zu 17 µmol DFO/g im Vergleich zu 0 µmol DFO/g des unbehandelten PU und 0.8 µmol DFO/g des nicht-aktivierten PU/DFO bestimmt.

Beispiel 5 - Immobilisierung von DFO an eine Polyurethanfolie

Ein 30 $cm^2$ großes Stück einer Polyurethanfolie (PU-Folie) mit einer Dicke von 30 µm und einem Gewicht von 130 mg wurde in einen Glaskolben gegeben und mit 20 ml absolutem Aceton überschichtet. Unter Rühren wurden 30 mg (185 µmol) CDI in 20 ml absolutem Aceton innerhalb von 5 min zugetropft und bei 50°C für 30 min unter Rückfluß gehalten. Nach dem CDI-Aktivierungsschritt wurde das Acetongemisch abgegossen und die aktivierte Folie mehrmals mit Aceton gewaschen und unverzüglich mit 120 mg (183 µmol) DFOM gelöst in 50 ml Bicarbonatpuffer (100 mM, pH 8.5) versetzt. Nach 12 h wurde die PU-Folie aus der Reaktionsmischung genommen und mehrfach mit deionisiertem Wasser gewaschen bis der Farbnachweis auf DFO mit Eisensulfatlösung negativ war. Die PU-DFO-Folie wurde an der Luft getrocknet und in einer verschlossenen Glasschale aufbewahrt.

Funktionalitäts-Assay des immobilisierten DFO mit Eisen

Durch Komplexbildung von Eisen mit gelöstem DFO entsteht im Verhältnis 1:1 der intensiv orange gefärbte DFO-Eisen-III-Komplex Ferrioxamin (P.E. Hallaway et al., PNAS 1989; 86: 10108). Die Inkubation der DFO-gekoppelten Matrices mit 5 mM Eisensulfat- (pH 5.0) oder Eisenchlorid-Lösung (pH 2.2) führt innerhalb weniger Minuten zu einer Orangefärbung der Verbandsbaumwolle, wobei die Probe mit Eisensulfat-Lösung langsamer färbt als die mit Eisenchlorid-Lösung. Dieser Effekt ist auf eine Ferroxidase-Eigenschaft des DFO-Moleküls zurückzuführen, die vor der Komplexierung des Eisens $Fe^{2+}$ zu $Fe^{3+}$ oxidiert (J.F. Goodwin und C.F. Whitten, Nature 1965; 205: 281).

Nach Stehenlassen über Nacht kam es zu einer Farbintensivierung der nach den obigen Beispielen hergestellten Muster. Für die visuelle Charakterisierung der DFO-gekoppelten Proben wurden je 100 mg an DFO-gekoppelter Verbandsbaumwolle, reine Verbandsbaumwolle und nichtaktivierte Verbandsbaumwolle, die mit DFOM-Lösung behandelt wurde, wie oben beschrieben, mit je 5 ml einer 5 mM Eisensulfat- bzw. Eisenchlorid-Lösung in 50-ml Plastikschraubgefäßen (Greiner, Nürtingen) für 18 h in einem Überkopfschüttler (Heidolph, Kelheim) inkubiert und die sich eingestellten Färbungen ausgewertet.

Methoden zur Quantifizierung des gesamten immobilisierten DFO

- Spektroskopische Quantifizierung in Lösung
  Durch Ermittlung der Konzentration an gelöstem DFO in der Kopplungslösung vor und nach der Reaktion und in den vereinigten Waschlösungen kann durch Differenzrechnung die Menge an immobilisiertem DFO bestimmt werden. Dazu wird ein Aliquot der DFO-Lösung mit einem 10-fachen Überschuß an $FeCl_3$ in die eisengebundene Form, Ferrioxamin, gebracht. Nach einer Inkubationszeit von 30 min wird die Absorption bei 430 nm in einem Uvikon 943 Photometer (Kontron, Neu. farn) gemessen. Die Errechnung der DFO-Konzentration erfolgt über das Lambert-Beersche Gesetz ($E = \varepsilon \cdot c \cdot d$; E = Extinktion [-], c = Konzentration an DFO [mol/l], d = Küvettenschichtdicke [cm] und ε = spezifischer molarer Extinktionskoeffizient von Ferrioxamin 2300 $M^{-1} \cdot cm^{-1}$). Die Sättigung des gelösten DFO mit Eisen ist ebenso mit einer Eisensulfatlösung zu erreichen (P.E. Hallaway et al., PNAS 1989; 86: 10108). Hier muß jedoch über einen längeren Zeitraum inkubiert werden, damit sich der anfänglich gebildete DFO-Fe-II-Komplex in den stabileren DFO-Fe-III-Komplex umwandeln kann. Um eine genaue und reproduzierbare Bestimmung der DFO-Konzentration zu erreichen, ist es erforderlich, daß alle zu messenden DFO-Lösungen eine gleiche Pufferzusammensetzung aufweisen.

- Quantifizierung mit HPLC
  Die Konzentrationsbestimmungen an DFO wurden mittels einer Nucleosil C18-reversed-phase Säule (Macherey & Nagel, Düren) der Dimension 125 cm x 4 mm und einer HPLC-Anlage von Pharmacia (Pharmacia LKB, Uppsala), Pumpe Model 2249 und Monitor Model VWM 2141, bei einer Detektion von 430 nm durchgeführt. Zur Kalibrierung wurden verschiedene DFOM-Standards (10 und 1 mM) in Bicarbonatpuffer (25 mM, pH <6) mit je einem 20 %-igen Überschuß einer Eisenchloridlösung versetzt und nach 30 min Inkubation bei einer Flußrate von 1 ml/min auf die mit Kaliumphosphatpuffer (30 mM, pH 3.5) äquilibrierte Säule gegeben. Nach Starten eines linearen Gradienten, von 0 auf 25% Acetonitril innerhalb von 9 min, eluierte Ferrioxamin bei 8.7 min. Die zu messenden DFO-Proben wurden analog vorbereitet. Zur Berechnung der gebundenen DFO-Menge wurde von der Kopplungskonzentration (10 mM) die DFO-Konzentration des Überstandes nach der Kopplungsreaktion, sowie die DFO-Konzentration in den vereinigten Waschlösungen abgezogen.

Methode zur Quantifizierung des eisenbindungsaktiven immobilisierten DFO

Stücke (50 bis 100 mg) von mit DFO-gekoppelter Verbandsbaumwolle bzw. DFO-gekoppeltem Polyurethan wurden in Schraubgefäßen aus Plastik mit je 10 bis 40 ml einer Lösung aus Eisensulfat (0.2 mM), $CaCl_2$ (1mM), $MgCl_2$ (1 mM) und NaCl (155 mM) über einen Zeitraum von 24 h im Überkopfschüttler inkubiert. Die zeitliche Abnahme des Eisens im Überstand wurde mit Hilfe des MPR 3 Fertigassays für Eisen (Boehringer, Mannheim) spektroskopisch bei 562 nm bestimmt. Mindestens 3 Muster einen Typs wurden gemessen, der Überstand wurde jeweils einer Dreifachbestimmung unterzogen. Nicht durch DFO komplexiertes Eisen wurde durch Ferrozin (3-(2-Pyridyl)-5,6-diphenyl-1,2,4-triazin-p,p'-disulfonsäure) unter Ausbildung eines violetten Komplexes (mit einem Absorptionsmaximum bei 562 nm und einem molaren Extinktionskoeffizienten von 29000 $M^{-1}cm^{-1}$) gebunden. Der Eisen-Ferrozin-Komplex besitzt einen um den Faktor zehn höheren Extinktionskoeffizienten im Vergleich zum Eisen-DFO-Komplex, was eine sehr empfindliche Nachweisgrenze von <10 µM Eisen ermöglicht. Als Kontrolle diente reines Eisensulfat, unbehandelte Verbandsbaumwolle (bzw. unbehandeltes Polyurethan) und nichtaktivierte Verbandsbaumwolle (nicht-aktiviertes Polyurethan), die entsprechend den Kopplungsbedingungen behandelt wurden. Über die Differenz der Eisenmenge vor und nach Inkubation mit den DFO-Mustern konnte auf die immobilisierte DFO-Menge geschlossen werden.

Methode zur Freisetzung von Eisen aus immobilisiertem DFO-Eisen (DFO-Fe)

Diese Methode basiert auf dem von T. Emery (Biochemistry 1986; 25: 4629) beschriebenen Verfahren und dient dem Nachweis von durch DFO komplexiertem Eisen. Dazu wurde an eine Matrix immobilisiertes DFO mit einer Eisenchlorid-Lösung (10 mM) für 10 min bei Raumtemperatur inkubiert und vollständig in die eisengebundene Form überführt. Danach wurde ungebundenes Eisen aus der Matrix mit 20 mM Acetatpuffer (pH 5.4) gewaschen bis der Eisennachweis des Waschpuffers mit Ferrozin keine Färbung mehr erkennen ließ. Das durch DFO komplexierte Eisen-III wurde schließlich durch 20 mM Ascorbat und 20 mM Gallium-III-nitrat in 100 mM Acetatpuffer (pH 5.4) aus dem immobilisierten DFO herausgedrängt, unter Reduktion des Eisens und Bindung von Ga-III durch DFO. Das somit freigesetzte Eisen-II wurde durch den Komplexbildner Ferrozin (4 mM) als Eisen-II-Komplex gebunden und die violettgefärbte Lösung bei 562 nm quantitativ bestimmt. Von dem gemessenen Eisenwert war jeweils der Wert der Kontrolle der ungekoppelten Matrix abzuziehen.

Methode zur Messung der in-vitro Eisenaufnahme aus Serum und Wundflüssigkeit

Frisches Spenderblut, abgefüllt in mit Heparin beschichteten Plastikröhrchen, wurde in Plasma und Serum durch Zentrifugation für 5 min bei 10000 rpm in einer Zentrifuge getrennt. Ein ml der Serumprobe wurde in ein Microtube (Eppendorf-Gefäß) pipettiert und auf 37°C temperiert. Danach wurden 0,1 µM freies DFOM, jeweils 10 mg Verbandsbaumwolle, DFO-gekoppelte Verbandsbaumwolle, PU und DFO-PU dazugegeben und in einem temperierten Schüttelgerät für bis zu 24 h inkubiert. Zu verschiedenen Zeitpunkten wurde die Gesamteisenmenge in der Serumprobe mit Hilfe des MPR 3 Eisenkits von Boehringer Mannheim bestimmt. Dabei wurde der verbleibende Gehalt an nichtgebundenem Eisen bestimmt, indem Eisen-III durch eine Detergenzmischung im schwach sauren pH-Bereich von Transferrin gelöst und mit Ascorbat zu Eisen-II reduziert wurde. Der mit Ferrozin gebildete Farbkomplex wurde spektroskopisch quantifiziert.

Beispiel 6 - Immobilisierung von SOD oder Katalase an Cellulose

Die verschiedenen Immobilisierungsreaktionen von Superoxid-Dismutase (SOD, EC 1.15.1.1) und Katalase (KAT, EC 1.11.1.6) wurden mit folgenden Enzymen durchgeführt: Cu,Zn-SOD aus bovinen Erythrozyten (Boehringer Mannheim), Fe-SOD und Mn-SOD aus *E. coli* (Sigma, München), Cu,Zn-SOD aus Meerretich (Sigma, München), SOD aus Hefe mit dem Handelsnamen Dismutin-BT® (Pentapharm, Basel) und Katalase aus bovinen Erythrozyten (Boehringer Mannheim). Dazu wurde die medizinische Verbandsbaumwolle (Gazomull®, Beiersdorf AG), wie bereits oben beschrieben, gekocht und mit CDI aktiviert. Stücke (0.5 g) der CDI-aktivierten Verbandsbaumwolle wurden in 50-ml-Plastikschraubgefäßen (Greiner, Nürtingen) mit 20 ml Bicarbonatpuffer (100 mM, pH 8.5) mit Enzymlösungen von 1 bis 50 mg/ml unter Schütteln in einem Überkopfschüttler (Heidolph, Kehlheim) bei Raumtemperatur für 16 h inkubiert. Die Überstände der Kopplungslösungen wurden dann in neue Plastikschraubgefäße überführt und für die Analyse bei 4°C aufbewahrt. Danach erfolgte dreimaliges Waschen mit je 30 ml Bicarbonatpuffer (100 mM, pH 8.5) und abschließend mit 30 ml Bicarbonatpuffer (100 mM, pH 8.5) und Kochsalz (500 mM), um nichtgebundenes Protein aus der Verbandsbaumwolle zu entfernen. Der Kopplungs- und Waschpuffer für Katalase wurde auf einen pH-Wert von 8.0 eingestellt. Restliche, noch nicht abreagierte Imidazolyl-Gruppen der Cellulose wurden durch Inkubation in Plastikschraubgefäßen mit 25 ml Glycin (200 mM) in Bicarbonatpuffer (100 mM, pH 8.5) bei Raumtemperatur im Überkopfschüttler für 16 h blockiert. Die so behandelte Cellulose wurde danach fünfmal mit je 30 ml Bicarbonatpuffer (100 mM, pH 8.5), dann zweimal mit 30 ml 0.9 %-iger Kochsalzlösung und dreimal mit 30 ml deionisiertem Wasser (Millipore, Bedford) gewaschen. Abschließend wurde die mit Enzym gekoppelte Cellulose in halbgeöffneten Petrischalen unter dem Abzug für 2 h luftgetrocknet, in Cellophanbeutel verpackt und bei 4°C gelagert.

Beispiel 7 - Immobilisierung von SOD und KAT an einen Celluloseschwamm

500 mg eines gepreßten Cellulosestücks (Cellspon®, Cellomeda Oy, Turku) mit einer Dicke von 2 mm wurden 30 min in 120 mM Bicarbonatpuffer gekocht und anschließend mit absolutem Aceton entwässert und im Vakuum getrocknet. Die Aktivierung mit 500 mg (3,08 mmol) CDI in 50 ml absolutem Aceton wurde in einem 100 ml Glaskolben bei 60°C für 60 min unter Rückfluß durchgeführt. Überschüßiges CDI wurde durch Waschen mit Aceton entfernt und dieses wiederum mit Aceton-Wasser-Transferbädern (s. o.) und durch Trocknen an der Luft. Die Kopplung mit 250 mg (7,7 µmol) SOD bzw. 300 mg (1,2 µmol) KAT erfolgte in 40 ml Bicarbonatpuffer (100 mM, pH 7,7) in 50-ml-Schraubgefäßen (Greiner, Nürtingen) unter intensiver Durchmischung. Nach 18 h wurde die Kopplungsreaktion gestoppt und mehrmals nacheinander mit 100 mM und 500 mM Bicarbonatpuffer und abschließend mit Wasser gewaschen bis kein ungebundenes Enzym mehr nachweisbar war. Zuletzt wurde das mit Enzym gekoppelte Cellspon®-Stück an der Luft getrocknet und in Schraubgefäßen bei Raumtemperatur aufbewahrt.

Beispiel 8 - Immobilisierung eines anti-PDGF-Antiköpers an Cellulose

Ein 10 x 10 cm (2 g) großes Stück Verbandsbaumwolle (Gazomull®, Beiersdorf AG) wurde wie oben beschrieben mit CDI aktiviert und gewaschen. Anschließend wurden 7.5 mg eines monoklonalen anti-PDGF-Antikörpers, der aus einer Maushybridoma-Zellinie stammt (vgl. PCT/EP93/02295; Spalte 12, Zeilen 27-32) in 30 ml Bicarbonat-Puffer (100 mM, pH 8.5) gelöst und zu der CDI-aktivierten Verbandsbaumwolle gegeben. Der Antikörper erkennt spezifisch die B Kette des PDGF-Dimers. Nach 24 h Inkubation bei Raumtemperatur in einer auf einer Wippe (Tecnomara, Zürich) befindlichen Plastik-Petrischale erfolgte die Aufarbeitung der Kopplungsreaktion wie oben beschrieben. Abschließend wurde mehrmals mit Wasser gewaschen und die mit Antikörper gekoppelte Verbandsbaumwolle bei Raumtemperatur in halbgeöffneten Petrischalen unter dem Abzug für 2 h luftgetrocknet, in Cellophanbeutel verpackt und bei 4°C gelagert.

Die Menge des an der Verbandsbaumwolle gebundenen anti-PDGF-Antikörpers wurde durch Messung des Antikörpertiters im Überstand vor der Kopplung abzüglich des Titers nach der Kopplung und in den Waschlösungen durch einen Bindungsassay bestimmt. Als Kontrolle dienten verschiedene Konzentrationen an gelöstem anti-PDGF-Antikörper.

Dazu wurde PDGF-AB (50 µg/ml) in Natriumhydrogencarbonatpuffer (200 mM, pH 7.8) an eine Mikrotiterplatte (Nunc, Wiesbaden) gebunden (50 µl pro Vertiefung), mit 1 %-iger BSA-Lösung (Bovine Serum Albumin Fraction V, Boehringer Mannheim, Bestellnummer 775 860) in TBS-Puffer (50 mM TRIS, 150 mM NaCl, pH 7.5, Carl Roth Karlsruhe) geblockt und nach dreimaligem Waschen mit TBS-Puffer mit 0.1 % Tween®20 (Poly(oxyethylen)$_n$-Sorbitan-Monolaurat, Bestellnummer 1 332 465, Boehringer Mannheim) (50 mM Tris, 150 mM NaCl, pH 7.5, Carl Roth, Karlsruhe) mit 200 µl der entsprechenden Antikörperlösungen für zwei Stunden bei Raumtemperatur inkubiert. Danach wurde wieder dreimal mit TBS-Puffer mit 0.1 % Tween®20 gewaschen und anschließend mit einem Ziege-anti-Maus-IgG-(H+L)-Meerrettichperoxidase-Konjugat (Bio-Rad, München, Bestellnummer 170 6516) als sekundärer Antikörper in einer Verdünnung von 1 : 1000 für weitere zwei Stunden inkubiert. Nach dreimaligem Waschen mit TBS-Puffer und einmaligem Waschen mit Natriumphosphatpuffer (20 mM, pH 6.8) wurde mit 0.7 mg/ml 5-Aminosalicylsäure (Sigma, München) und 0.5 µl/ml 30 %-iger Wasserstoffperoxidlösung (Merck, Darmstadt) für 15 Minuten gefärbt und mit 100 µl je Vertiefung 2 N Natronlauge gestoppt. Die Auswertung erfolgte mit einem ELISA-Reader (Dynatech MR 5000, Denkendorf) bei 450 nm und ergab Kopplungsausbeuten von 100 bis 200 µg anti-PDGF-Antikörper/g.

Beispiel 9 - Immobilisierung von Trypsininhibitor aus Sojabohnen an Cellulose

Ein 10 x 10 cm (2 g) großes Stück Verbandsbaumwolle wurde wie oben beschrieben mit CDI aktiviert und gewaschen. Anschließend wurden 20 mg Trypsininhibitor aus Sojabohnen (Typ I-S, Sigma, München) in 20 ml Bicarbonatpuffer (100 mM, pH 8.5) gelöst zu der aktivierten Verbandsbaumwolle gegeben und auf einer Wippe (Tecnomara, Zürich) in einer Plastik-Petrischale bei Raumtemperatur für 24 h inkubiert. Die Aufarbeitung der Kopplungsreaktion erfolgte wie oben beschrieben. Abschließend wurde mit Wasser gewaschen. Die mit Proteaseinhibitor gekoppelte Cellulose wurde in halbgeöffneten Petrischalen unter dem Abzug für 2 h bei Raumtemperatur getrocknet, in Cellophanbeutel verpackt und bei 4°C gelagert.

Methode zur Bestimmung von immobilisiertem Protein

Zum qualitativen Nachweis von immobilisierter SOD auf Verbandsbaumwolle wurde diese mit Hilfe der Immunfluoreszenzmikroskopie (IFM) untersucht. Alle Inkubationsschritte wurden bei Raumtemperatur durchgeführt. Die Antikörper-Verdünnungen und Waschschritte wurden sämtlich mit PBST-Puffer (Phosphat gepufferte Kochsalzlösung mit 0.1% Tween®20) vorgenommen. Stücke einer Größe von je 1 $cm^2$ der unbehandelten sowie der mit SOD kovalent verknüpften Verbandsbaumwolle wurden mit 3%igem Ziegen-Normalserum (DAKO, Nr. X 0907) in PBS (Phosphat gepufferte Kochsalzlösung) für 1 h geblockt und zweimal gewaschen. Anschließend wurden die Verbandsbaumwoll-Muster mit einem Kaninchen-anti-SOD-Antikörper (Biotrend, Nr. 100-4191) in einer Verdünnung von 1 : 100 eine weitere Stunde inkubiert. Nach vier Waschschritten folgte eine einstündige Inkubation mit fluoreszenzmarkiertem Ziege-anti-Kaninchen-IgG(H+L)∗Cy2 (Dianova, Nr. 111-225-003) in einer Verdünnung von 1 : 200 unter Lichtausschluß. Die Präparate wurden sechsmal gewaschen, auf Deckgläser überführt und diese mit einer Lösung von 30 mg 1,4-Diazabicyclo[2.2.2]octan (DABCO; Sigma, Nr. D-2522) pro ml MOWIOL Fixiersubstanz (Calbiochem, Nr. 475904) auf Objektträgern fixiert. Die mikroskopische Untersuchung erfolgte mit einem Zeiss Axioskop im Phasenkontrast- und Fluoreszenzmodus (FITC-Filter).

Methode zur Quantifizierung von gelöstem und immobilisiertem Protein

Zur Konzentrationsbestimmung von gelöstem und immobilisiertem Protein wurde der BCA-Protein-Assay von Pierce (Rockford, Illinois) verwendet. Dabei werden Kupfer-II-Ionen im alkalischen Milieu durch Protein zu Kupfer-I-Ionen, entsprechend der Biuret-Reaktion, reduziert (R.E. Brown et al., Analytical Biochemistry 1989; 180: 136). Die Menge an reduziertem Kupfer ist innerhalb einer Konzentrationsspanne von 20 bis 2000 µg/ml proportional zur Proteinkonzentration. Das lösliche Kupferdetektionsreagenz Bichinolin-4-carbonsäure (BCA) reagiert dann mit reduziertem Kupfer-I-Ion zu einem purpur gefärbten Komplex, dessen Absorptionsmaximum bei 562 nm liegt.

Zur Bestimmung von an Verbandsbaumwolle immobilisiertem Protein wurde die von T.M. Stich (Analytical Biochemistry 1990; 191: 343) publizierte Methode weitgehend übernommen. Andere Proteinbestimmungsmethoden wie die von M.M. Bradford und O.H. Lowry zeigen Bindung des Chromophors an die Matrix. Die Durchführung des BCA-Proteinassays mit an Verbandsbaumwolle immobilisiertem Protein wurde in einer Gewebekulturplatte (Greiner, Nürtingen) durchgeführt. Die Verbandsbaumwolle mit gekoppeltem Protein wurde in Stücken zu 20, 30 und 40 mg in den Kavitäten der Gewebekulturplatte in einem Gesamtvolumen von 1.68 ml (80 µl Probe und 1.6 ml BCA-Lösung) für 45 bis 60 min bei 37°C oder bei 60°C inkubiert. Zur Konzentrationsbestimmung des immobilisierten Proteins wurde das jeweils gelöste Protein in einer Verdünnungsreihe von 125 bis 1500 µg/ml als Standard mitinkubiert. Abschließend wurde die Gewebekulturplatte bei 562 nm in einem Microplate Reader Modell MR 5000 (Dynatech, Denkendorf) gemessen und ausgewertet.

Methode zur Aktivitätsbestimmung von gelöster SOD

Für die Messung der Aktivität der verschiedenen SOD-Enzyme wurde der Biotech SOD-525-Assay (OXIS International S.A., Frankreich) eingesetzt. Dieser Assay basiert auf dem durch SOD verursachten Anstieg der Geschwindigkeitskonstante der Autooxidationsreaktion des Catechols 5,6,6a,11b-Tetrahydro-3,9,10-trihydroxybenzo[c]fluoren (BXT-01050) in wässriger alkalischer Lösung (C. Nebot et al., Analytical Biochemistry 1993; 214: 442). Diese Autooxidationsreaktion erzeugt ein Chromophor mit einer maximalen Absorption bei 525 nm.

Der Assay wurde in einem luftgesättigten Puffer mit 50 mM 2-Amino-2-methyl-1,3-propandiol, 0.1 mM Diethylentriaminpentaessigsäure und 3 mM Borsäure, pH 8.8, bei 37°C durchgeführt. Kinetiken wurden bei 525 nm innerhalb einer Minute nach Zugabe von BXT-01050 gemessen. Die SOD-Aktivität wurde durch Ermittlung des Verhältnisses $V_s/V_c$ der Geschwindigkeitskonstanten der Autooxidationsreaktion bestimmt, wobei die Geschwindigkeitskonstanten V in Gegenwart ($V_s$) und in Abwesenheit ($V_c$) der Probe gemessen wurden. Eine SOD-Einheit (SOD-525) wird definiert als die Aktivität, die den Hintergrund der Autooxidationsreaktion verdoppelt, das heißt $V_s/V_c = 2$. Die Aktivität der Volumenaktivität (SOD) in SOD-525 Units/ml erfolgt nach Ermittlung von $V_s/V_c$ über folgende Formel (vom Hersteller angegeben):

$$(SOD) = \frac{0.93\ (V_s/V_c - 1)}{1.073 - 0.073\ (V_s/V_c)}$$

Methode zur Aktivitätsbestimmung von gelöster KAT

Katalase (EC 1.11.1.6, Boehringer Mannheim) katalysiert die Spaltung von Wasserstoffperoxid ($H_2O_2$) in Sauerstoff und Wasser. Die Aktivitätsbestimmung des gelösten Enzyms erfolgt mit einem spektrophotometrischen Assay modifiziert nach R.F. Beers und I.W. Sizers (J. Biol. Chem. 1952; 195: 133). Die Extinktionsabnahme bei 240 nm korreliert hierbei mit der durch Katalase hervorgerufenen Abnahme von Wasserstoffperoxid im Reaktionsgemisch.

In eine Quartzküvette (Hellma, Jena) wurden 3 ml Phosphatpuffer (50 mM, pH 7.0) (Referenzposition) bzw. 3 ml eines mit $H_2O_2$ versetzten Phosphatpuffers (0,05 mM $H_2O_2$, pH 7) (Probe) mit je 0,05 ml der Katalase-haltigen Probe gegeben. Dabei wurde die Wasserstoffperoxidkonzentration in dem $H_2O_2$-haltigen Puffer durch Extinktionsmes-

sung bei 240 nm kontrolliert, deren Absorption bei 0.500 ± 0.01 liegt. Nach Mischen der Komponenten in den Quartzküvetten (Schichtdicke 10 mm) wurde die Reaktion solange beobachtet, bis die Extinktion einen Wert von 0.450 erreicht hatte. Ab dann wurde mit einer Stoppuhr diejenige Zeitspanne erfaßt, die benötigt wurde, um die Extinktion auf 0.400 zu vermindern. Die Enzymkonzentration in der Küvette wurde dahingehend angepaßt, daß die hierfür benötigte Zeit 20 ± 2 Sekunden [s] betrug.

Die Errechnung der Volumenaktivität erfolgte nach folgender Formel:

$$\text{Volumenaktivität [U/ml]} = (17 \cdot 13) / \text{gestoppte Zeit [s]} \cdot 0.05$$

$$\text{Gesamtaktivität der Probe [U]} =$$

$$\text{Volumenaktivität [U/ml]} \cdot \text{Verdünnung} \cdot \text{Probenvolumen [ml]}$$

Eine Einheit (Unit, [U]) ist dabei definiert als diejenige Enzymaktivität, die 1 µmol Wasserstoffperoxid unter den gewählten Assaybedingungen (25°C, pH 7,0) pro Minute disproportioniert.

Methode zur Aktivitätsbestimmung von immobilisiertem Enzym, Antikörper und Protein

- an Cellulose immobilisierte SOD

  Die Aktivitätsbestimmung der immobilisierten SOD (vgl. L. Goldstein, Meth. Enzym., 1976; 44:397) erfolgte prinzipiell nach dem gleichen Assayprinzip, wie es zuvor für das gelöste Enzym beschrieben wurde. Es war eine apparative Anpassung des Meßvorgangs erforderlich. Anstelle der Quartzküvette wurde eine speziell konstruierte Quartz-Rührküvette (Hellma, Jena) verwendet, die durch ein auf dem Küvetten-Rundboden rührendes Magnetrührstäbchen (teflonbeschichtet, 8 x 3 mm, Hellma, Jena) während der Messung gut durchmischbar ist. Weiterhin wurde der passende Rührküvettenhalter mit zugehörigem Magnetrührwerk (Kontron, Neufahrn) mit variabel einstellbarer Geschwindigkeit verwendet. Über dem Magnetrührstäbchen wurde ein Muster der Verbandsbaumwolle (ca. 10 mg) mit immobilisierter SOD plaziert. Als Kontrolle diente ein ebenso schweres unbehandeltes medizinisches Baumwollstückchen in der Referenzposition. Die Baumwollgaze wurden durch eine speziell konstruierte Einlage von der darüberliegenden Flüssigkeit getrennt, so daß ein Aufschwimmen evtl. sich lösender Flusen verhindert wurde, der Austausch aller Reaktionsteilnehmer durch Mischen noch gewährleistet war. Der Strahlengang wurde durch den Einbau der Einlage nicht blockiert. Die Reaktion wurde durch Zugabe von BXT--01050 gestartet. Die Anpassung der Enzymmenge an die geforderten Mindest- bzw. Höchstautooxidationsraten erfolgte durch das Gewicht an zugebenem Muster. Die Berechnung der Aktivität des immobilisierten Enzyms erfolgte durch die Hersteller-Formel (s.o.). Das Ergebnis wurde in U-525-Einheiten je mg Mustereinwaage berechnet und auf 1 g Mustereinwaage normiert. Jedes Muster wurde mindestens einer 5-fach-Bestimmung unterzogen.

- an Cellulose immobilisierte Katalase

  Die Aktivitätsbestimmung erfolgte prinzipiell nach der bereits für die gelöste Katalase beschriebenen Methode (siehe oben). Die für die Aktivitätsbestimmungen immobilisierter Enzyme erforderliche apparative Anpassung des Meßsystems ist bereits oben (für SOD) beschrieben.

  Die Reaktion wurde durch Zugabe des Wasserstoffperoxid-haltigen Puffers gestartet. Die Anpassung der Enzymmenge an die geforderten Grenzwerte erfolgte durch das Gewicht an zugebenem Muster. Die Berechnung der Aktivität des immobilisierten Enzyms erfolgte durch folgende Formel:

  $$\text{Aktivität [U] je Einwaage Muster} = (17 \times 13) / \text{gestoppte Zeit [s]} \cdot \text{Einwaage Muster [mg]}$$

  Das Ergebnis wurde auf 1 g Muster hochgerechnet. Jedes Muster wurde mindestens einer 5-fach-Bestimmung unterzogen.

- an Cellulose immobilisierter anti-PDGF-Antikörper

  Die Aktivität des an Verbandsbaumwolle immobilisierten anti-PDGF-Antikörpers wurde durch Bindung von PDGF-AB Heterodimer bestimmt. Dazu wurden Proben an Verbandsbaumwolle von 10 bis 250 mg mit PDGF-AB (50 ng/ml) in physiologischer Kochsalzlösung für 30 min bei Raumtemperatur inkubiert. Danach wurden die Muster mehrfach gespült, und die verbleibende Konzentration an PDGF-AB wurde mit dem in PCT/EP93/02295 (Spalte 12, Zeilen 27-32) beschriebenen ELISA bestimmt.

- an Cellulose immobilisierter Trypsin-Inhibitor
  Die Aktivität des gekoppelten Trypsin-Inhibitors wurde über seine Fähigkeit, Trypsin in seiner Aktivität zu hemmen, bestimmt. Die Trypsin-Aktivitätsbestimmung erfolgte prinzipiell nach der Methode von G.W. Schwert und Y. Takenaka (Biochim. Biophys. Acta 1955; 16:570-575). Nach dieser Methode wird durch die Trypsin-Aktivität aus dem Substrat N-Benzoyl-L-argininethylester (BAEE) N-Benzyol-L-arginin freigesetzt.
  Die durch diese Reaktion entstehende Extinktionszunahme bei 253 nm wurde im vorliegenden Fall mit einem Spektralphotometer verfolgt (Uvikon 943, Kontron, Neufahrn).
  Verschieden schwere, mit Trypsin-Inhibitor gekoppelte Cellulosestückchen (10-50 mg) wurden abgewogen und in 2.8 oder 5.6 ml der Substratlösung gelegt. Die Herstellung der Substratlösung wurde wie in "Bestimmungsmethoden für Enzyme" (B. Stellmach, Steinkopff Verlag Darmstadt, 1988, S. 266) beschrieben durchgeführt. Die Mischung wurde auf 25°C temperiert und geschüttelt (GFL-Wasserbad 1083, GFL, Großburgwedel), und die Reaktion wurde durch Zugabe von 0.2 ml bzw. 0.4 ml einer Trpysinlösung (ca. 150 Units/ml, gelöst in 0.001 N HCl) gestartet. Dann wurden 0.5 ml des Überstandes einmal pro Minute für mindestens 5 Minuten in eine frische Quartzküvette überführt und die Extinktion bei 253 nm bestimmt. Der Küvetteninhalt wurde schnellstmöglich in die Reaktionsmischung zurückgegeben. Bei konstanter Extinktionszunahme pro Minute wurde die Messung beendet und die Trypsinaktivität nach der Berechnungsformel in der angegebenen Publikation von B. Stellmach errechnet:

$$\frac{E_{253}}{0.001 \cdot E_w} = \text{Einheiten/mg}$$

| | |
|---|---|
| $E_{253}$: | Extinktionszunahme bei 253 nm/min |
| 0.001: | 1 Einheit bedeutet eine Extinktionszunahme von 0.001/min |
| $E_w$: | Trypsin-Einwaage in mg/0.2 ml (bzw. 0.4 ml) eingesetzter Lösung |

Als Kontrollen dienten Cellulosemuster ohne Vorbehandlung und Muster ohne Aktivierung, jedoch mit Inhibitorbehandlung. Die Ergebnisse der Kontrollen wurden bei der Errechnung der Aktivität des immobilisierten Inhibitors berücksichtigt. Durch Aufnahme einer Standardgerade mit gelöstem Trypsin-Inhibitor war eine Abschätzung der immobilisierten Inhibitormenge möglich.

## Patentansprüche

**1.** Verwendung von Substanzen, die mit in Wundexsudat vorhandenen Störfaktoren, die den Wundheilungsprozeß behindern, wechselwirken, zur Herstellung von Wundauflagen, wobei die Störfaktoren aus der Gruppe bestehend aus suspendierten Zellen und Zellfragmenten sowie gelösten Bestandteilen ausgewählt sind, wobei die Wechselwirkung ein Binden, Komplexieren, Chelatisieren des Störfaktors oder eine chemische Reaktion mit dem Störfaktor umfaßt und wobei die Substanzen kovalent an ein Trägermaterial gebunden sind.

**2.** Verwendung nach Anspruch 1, dadurch gekennzeichnet, daß die gelösten Bestandteile aus der Gruppe bestehend aus Antigenen, Radikalen, Ionenn, Proteinen, Peptiden, Lipiden und freien Fettsäuren ausgewählt sind.

**3.** Verwendung nach Anspruch 1 oder 2, dadurch gekennzeichnet, daß die Substanz aus der Gruppe bestehend aus Antikörpern, Chelatoren, Enzyminhibitoren, Enzymen, Peptiden und anderen Proteinen ausgewählt ist.

**4.** Verwendung nach den Ansprüchen 1 bis 3, dadurch gekennzeichnet, daß das Trägermaterial ein polymeres Trägermaterial natürlichen oder synthetischen Ursprungs ist.

**5.** Verwendung nach Anspruch 4, dadurch gekennzeichnet, daß das polymere Trägermaterial aus der Gruppe bestehend aus Cellulose und deren Derivaten, Alginaten, Hyaluronsäure, Chitin, Chitosanen, Polysacchariden, Polyamiden, Polyestern, Polyolefinen, Polyacrylaten, Polyvinylalkoholen, Polyurethanen, Siliconen einschließlich deren Mischungen und Copolymeren ausgewählt ist.

**6.** Verwendung nach den Ansprüchen 1 bis 5, dadurch gekennzeichnet, daß die Wundauflage aus der Gruppe bestehend aus Verbänden, Verbandmull, Binden, Kompressen, Watten, Pflastern, Folien, Filmen, Hydrokolloidverbänden und Gelen ausgewählt ist.

7. Verwendung nach den Ansprüchen 1 bis 6, dadurch gekennzeichnet, daß die Störfaktoren Ionen sind und die mit den Störfaktoren wechselwirkende Substanz ein Chelator ist.

8. Verwendung nach Anspruch 7, dadurch gekennzeichnet, daß der Chelator aus der Gruppe bestehend aus Desferrioxamin, Diethylentriaminpentaessigsäure, N,N'-Bis-(o-hydroxybenzyl)-ethylendiamin-N,N'-diessigsäure, 1,2-Dimethyl-3-hydroxypyrid-4-on und 1,2-Dimethyl-3-hydroxyl-3-hydroxypyridin-4-on ausgewählt ist.

9. Verwendung nach Anspruch 8, dadurch gekennzeichnet, daß die Störfaktoren Eisen-III-Ionen sind und die mit den Störfaktoren wechselwirkende Substanz Desferrioxamin ist.

10. Verwendung nach den Ansprüchen 1 bis 6, dadurch gekennzeichnet, daß die Störfaktoren reaktive Sauerstoffradikale sind und die mit den Störfaktoren wechselwirkende Substanz ein Radikalfänger ist.

11. Verwendung nach Anspruch 10, dadurch gekennzeichnet, daß der Radikalfänger aus der Gruppe bestehend aus Superoxiddismutase, Katalase, Glutathion-Peroxidase, Myeloperoxidase und Enzym-Mimics oder einer anderen Kombination davon ausgewählt ist.

12. Verwendung nach den Ansprüchen 1 bis 6, dadurch gekennzeichnet, daß der Störfaktor eine Protease ist und die mit dem Störfaktor wechselwirkende Substanz ein Protease-Inhibitor ist.

13. Verwendung nach Anspruch 12, dadurch gekennzeichnet, daß der Protease-Inhibitor aus der Gruppe bestehend aus Antipain, Leupeptin, Cystain, Pepstatin, Diisopropylfluorophosphat, 4-(2-Aminoethyl)-phenylsulfonylfluorid, Phenylmethansulfonylfluorid, natürliche proteinogene Protease-Inhibitoren aus der Klasse der Tissue Inhibitors of Matrixmetalloproteinases, Aprotinin, Alpha-2-Antiplasmin, Alpha-2-Makroglobulin, Alpha-1-Antichymotrypsin, Sojabohnen-Trypsininhibitor und Alpha-1-Protease-Inhibitor ausgewählt ist.

14. Wundauflage, dadurch gekennzeichnet, daß an ein Trägermaterial Substanzen kovalent gebunden sind, die mit in Wundexsudat vorhandenen Störfaktoren, die den Wundheilungsprozeß behindern, wechselwirken, wobei die Störfaktoren aus der Gruppe bestehend aus suspendierten Zellen und Zellfragmenten sowie gelösten Bestandteilen ausgewählt sind und wobei die Wechselwirkung ein Binden, Komplexieren, Chelatisieren des Störfaktors oder eine chemische Reaktion mit dem Störfaktor umfaßt.

15. Wundauflage nach Anspruch 14, dadurch gekennzeichnet, daß die gelösten Bestandteile aus der Gruppe bestehend aus Antigenen, Radikalen, Ionen, Proteinen, Peptiden, Lipiden und freien Fettsäuren ausgewählt sind.

16. Wundauflage nach Anspruch 14 oder 15, dadurch gekennzeichnet, daß die Substanz aus der Gruppe bestehend aus Antikörpern, Chelatoren, Enzyminhibitoren, Enzymen, Enzym-Mimics, Peptiden und anderen Proteinen ausgewählt ist.

17. Wundauflage nach den Ansprüchen 14 bis 16, dadurch gekennzeichnet, daß das Trägermaterial ein polymeres Trägermaterial natürlichen oder synthetischen Ursprungs ist.

18. Wundauflage nach Anspruch 17, dadurch gekennzeichnet, daß das polymere Trägermaterial aus der Gruppe bestehend aus Cellulose und deren Derivaten, Alginaten, Hyaluronsäure, Chitin, Chitosanen, Polysacchariden, Polyamiden, Polyestern, Polyolefinen, Polyacrylaten, Polyvinylalkoholen, Polyurethanen, Siliconen einschließlich deren Mischungen und Copolymeren ausgewählt ist.

19. Wundauflage nach den Ansprüchen 14 bis 18, dadurch gekennzeichnet, daß die Wundauflage aus der Gruppe bestehend aus Verbänden, Verbandmull, Binden, Kompressen, Watten, Pflastern, Folien, Filmen, Hydrokolloidverbänden und Gelen ausgewählt ist.

20. Wundauflage nach den Ansprüchen 14 bis 19, dadurch gekennzeichnet, daß die Störfaktoren Ionen sind und die mit den Störfaktoren wechselwirkende Substanz ein Chelator ist.

21. Wundauflage nach Anspruch 20, dadurch gekennzeichnet, daß der Chelator aus der Gruppe bestehend aus Desferrioxamin, Diethylentriaminpentaessigsäure,N,N'-Bis-(o-hydroxybenzyl)-ethylendiamin-N,N'-diessigsäure, 1,2-Dimethyl-3-hydroxypyrid-4-on und 1,2-Dimethyl-3-hydroxyl-3-hydroxypyridin-4-on ausgewählt ist.

**22.** Wundauflage nach Anspruch 21, dadurch gekennzeichnet, daß die Störfaktoren Eisen-III-Ionen sind und die mit den Störfaktoren wechselwirkende Substanz Desferrioxamin ist.

**23.** Wundauflage nach den Ansprüchen 14 bis 19, dadurch gekennzeichnet, daß die Störfaktoren reaktive Sauerstoffradikale sind und die mit den Störfaktoren wechselwirkende Substanz ein Radikalfänger ist.

**24.** Wundauflage nach Anspruch 23, dadurch gekennzeichnet, daß der Radikalfänger aus der Gruppe bestehend aus Superoxiddismutase, Katalase, Glutathion-Peroxidase, Myeloperoxidase und Enzym-Mimics oder einer Kombination davon ausgewählt ist.

**25.** Wundauflage nach den Ansprüchen 14 bis 19, dadurch gekennzeichnet, daß der Störfaktor eine Protease ist und die mit dem Störfaktor wechselwirkende Substanz ein Protease-Inhibitor ist.

**26.** Wundauflage nach Anspruch 25, dadurch gekennzeichnet, daß der Protease-Inhibitor aus der Gruppe bestehend aus Antipain, Leupeptin, Cystain, Pepstatin, Diisopropylfluorophosphat, 4-(2-Aminoethyl)-phenylsulfonylfluorid, Phenylmethan-sulfonylfluorid, natürliche proteinogene Protease-Inhibitoren aus der Klasse der Tissue Inhibitors of Matrixmetalloproteinases, Aprotinin, Alpha-2-Antiplasmin, Alpha-2-Makroglobulin, Alpha-1-Antichymotrypsin, Sojabohnen-Trypsininhibitor und Alpha-1-Protease-Inhibitor ausgewählt ist.

**27.** Wundauflage nach den Ansprüchen 14 bis 26, dadurch gekennzeichnet, daß sie zusätzlich wundheilungsfördernde Substanzen enthält.

**28.** Wundauflage nach Anspruch 27, dadurch gekennzeichnet, daß die wundheilungsfördernden Substanzen Wachstumsfaktoren sind.

**29.** Wundauflage nach den Ansprüchen 14 bis 28, dadurch gekennzeichnet, daß sie Feuchtigkeit aufnimmt.

**30.** Verfahren zur Herstellung einer Wundauflage nach den Ansprüchen 14 bis 29, dadurch gekennzeichnet, daß man die mit dem Störfaktor wechselwirkende Substanz durch chemische Reaktion kovalent an das Trägermaterial bindet.

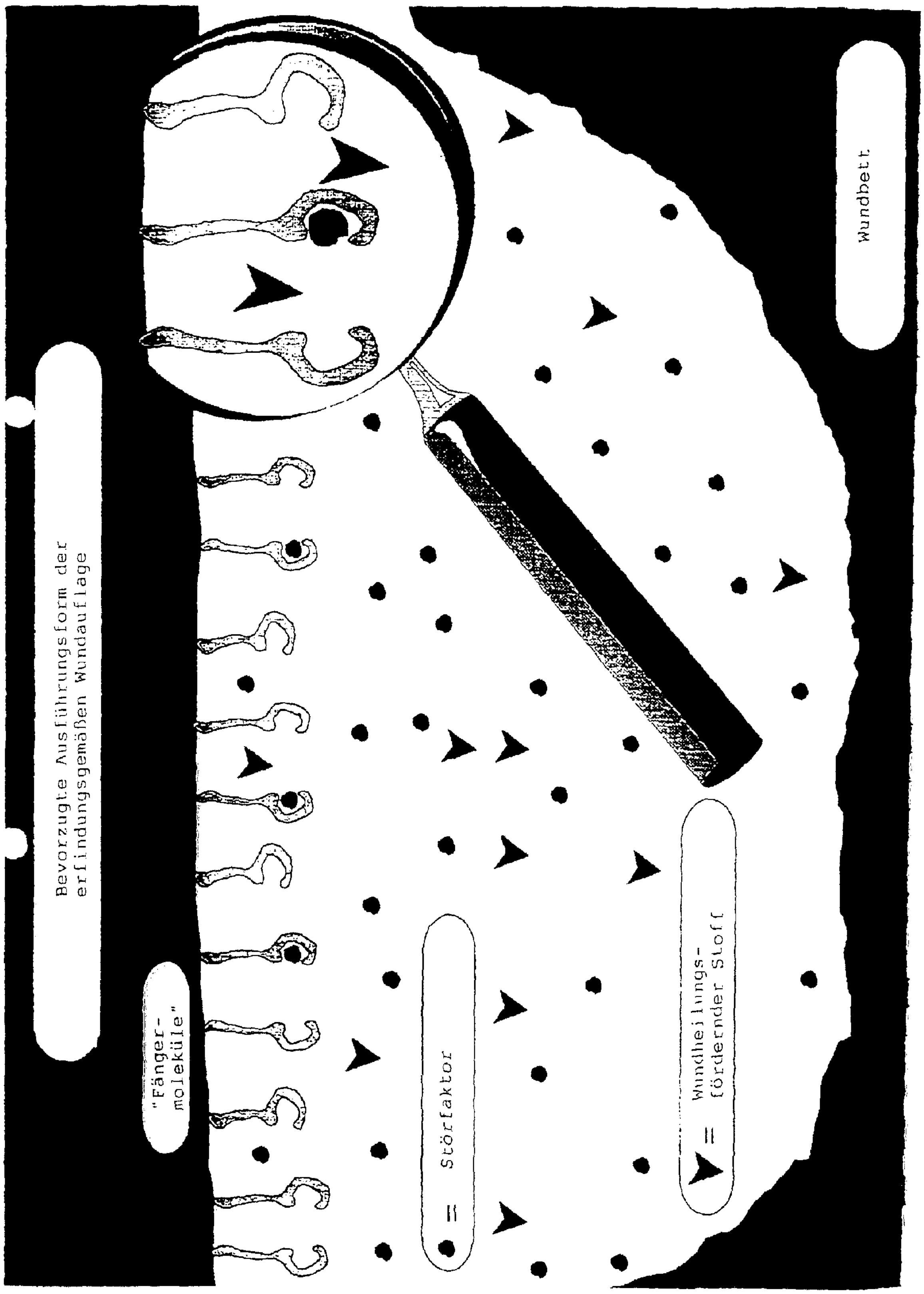
Bevorzugte Ausführungsform der
erfindungsgemäßen Wundauflage
"Fänger-
moleküle"
= Störfaktor
= Wundheilungs-
fördernder Stoff
Wundbett

Fig. 1

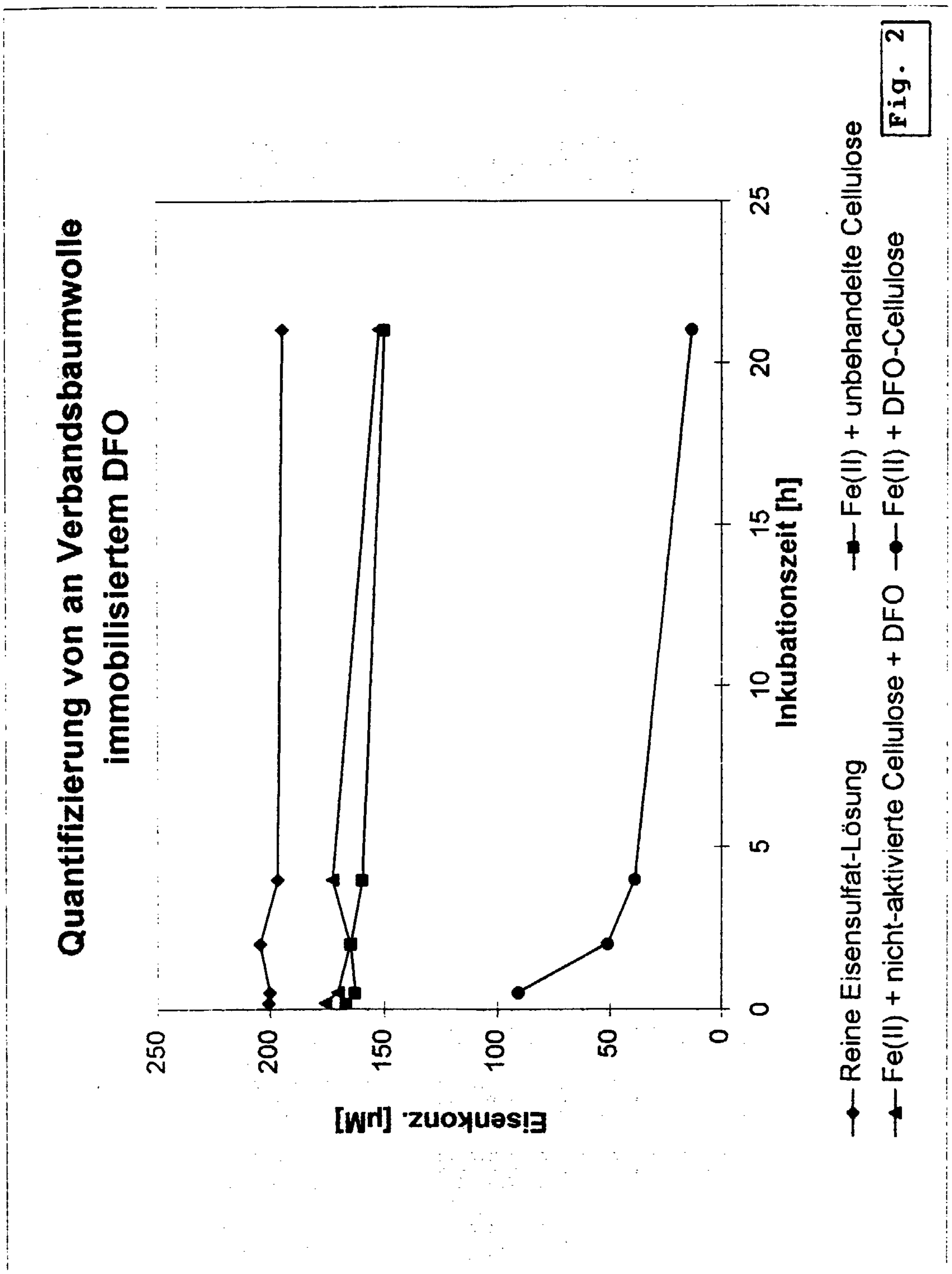
Quantifizierung von an Verbandsbaumwolle immobilisiertem DFO
Eisenkonz. [µM]
250
200
150
100
50
0
0
5
10
15
20
25
Inkubationszeit [h]
Reine Eisensulfat-Lösung
Fe(II) + unbehandelte Cellulose
Fe(II) + nicht-aktivierte Cellulose + DFO
Fe(II) + DFO-Cellulose

Fig. 2

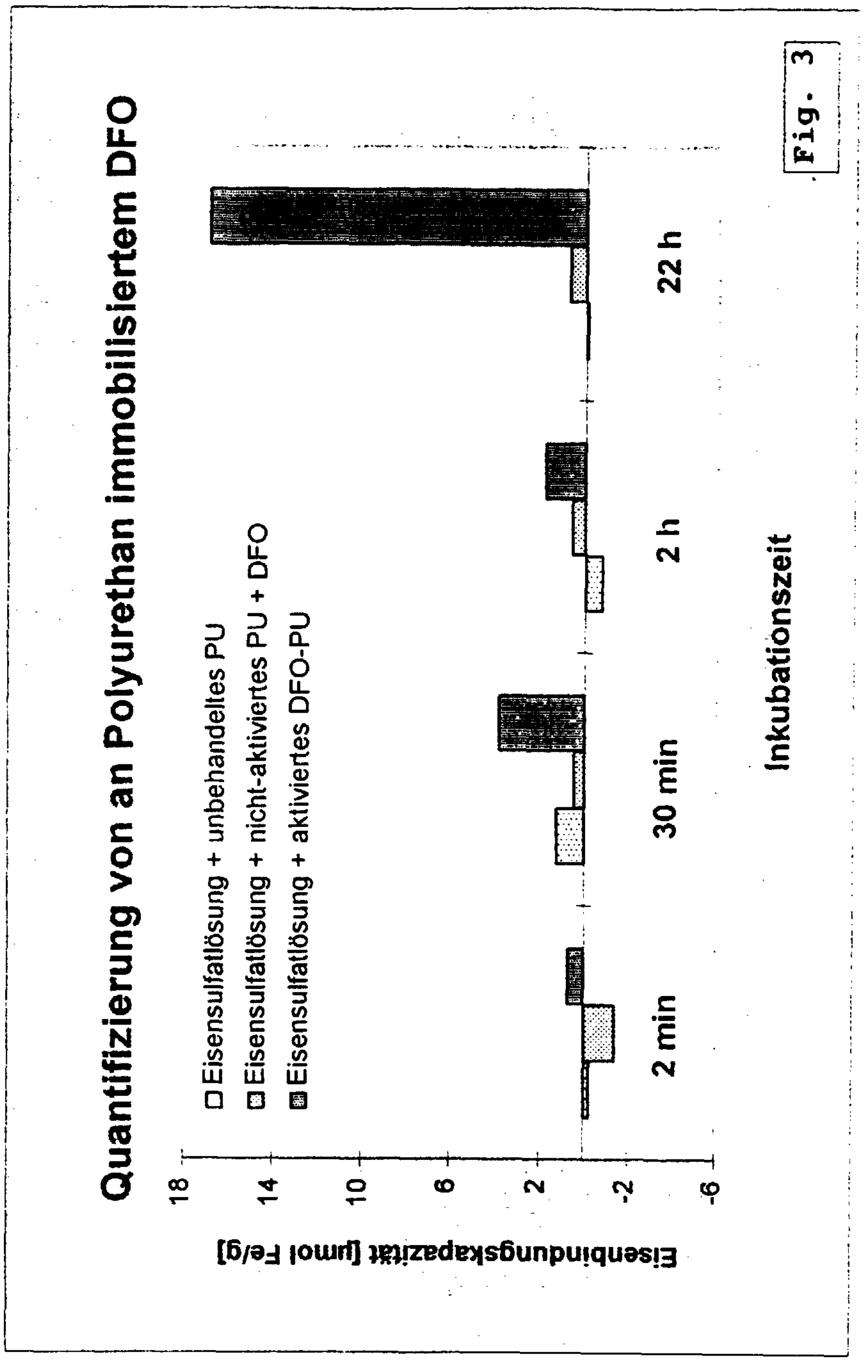
Quantifizierung von an Polyurethan immobilisiertem DFO
Eisensulfatlösung + unbehandeltes PU
Eisensulfatlösung + nicht-aktiviertes PU + DFO
Eisensulfatlösung + aktiviertes DFO-PU
Eisenbindungskapazität [µmol Fe/g]
18
14
10
6
2
-2
-6
2 min
30 min
2 h
22 h
Inkubationszeit

Fig. 3

Fig. 4

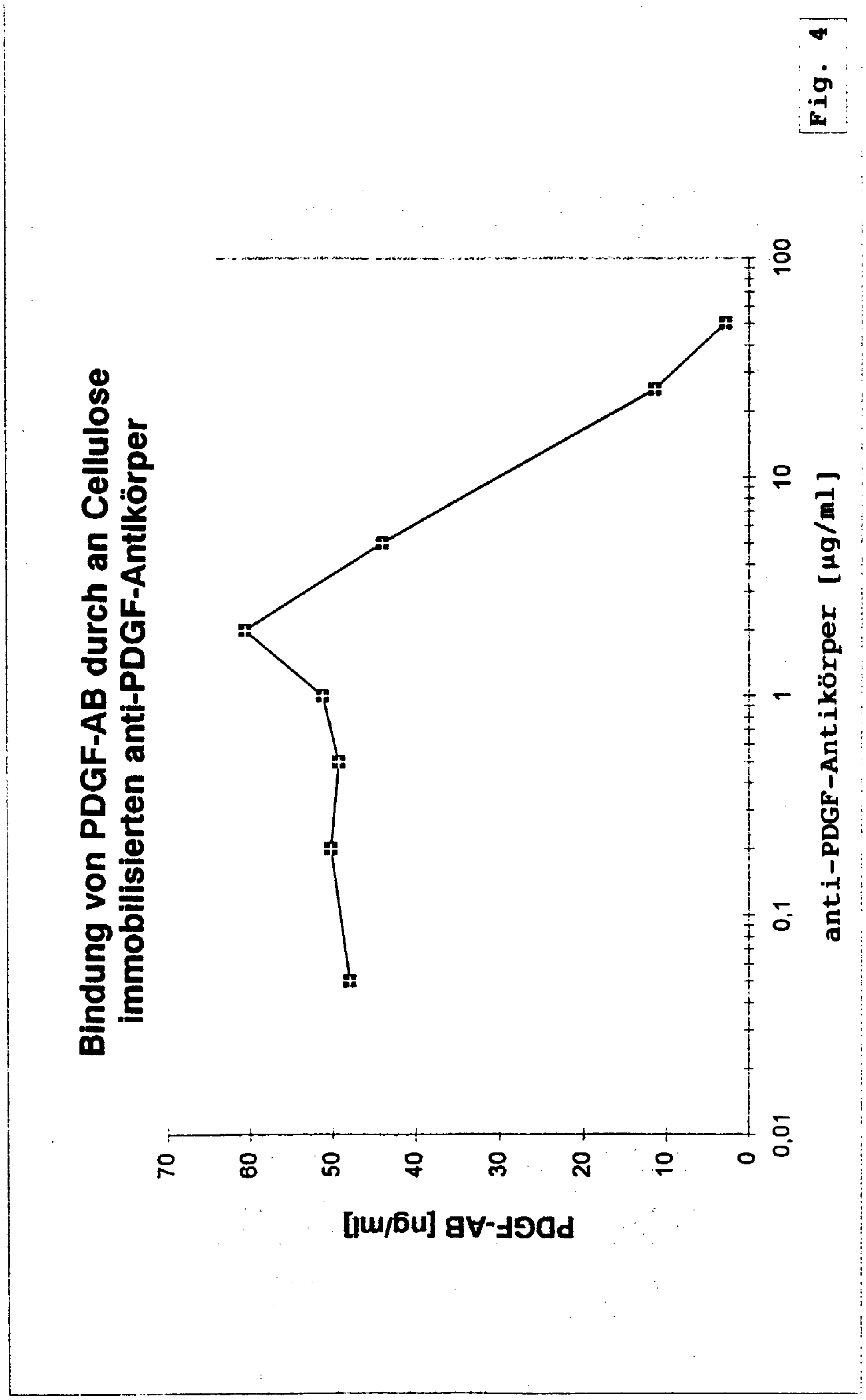
Bindung von PDGF-AB durch an Cellulose
immobilisierten anti-PDGF-Antikörper
PDGF-AB [ng/ml]
70
60
50
40
30
20
10
0
0,01
0,1
1
10
100
anti-PDGF-Antikörper [µg/ml]